# EUROPEAN PATENT APPLICATION

(11) **EP 2 868 328 A1**
(43) Date of publication of application: **06.05.2015**
(21) Application number: 13005166.7
(22) Date of filing: 31.10.2013
(51) Int. Cl.: A61K 39/395, C07K 16/24, A61P 37/06

(54) **IL-3 blockade in systemic lupus erythematosus and multiple sclerosis**

(71) Applicant: Universitätsklinikum Regensburg, 93053 Regensburg (DE)
(72) Inventor: Mack, Matthias, 93051 Regensburg (DE); Renner, Kerstin, 93096 Köfering (DE); Brühl, Hilke, 93051 Regensburg (DE)
(74) Representative: Engelhard, Markus

(57) **Abstract**

The present invention relates to anti-IL-3 antibodies or IL-3 binding fragments thereof for use in the treatment of an autoimmune disease selected from the group consisting of systemic lupus erythematosus and multiple sclerosis, and to pharmaceutical compositions comprising such an antibody or antibody fragment.

SEQUENCE LISTING

<110> Universitätsklinikum Regensburg

<120> IL-3 Blockade in Systemic Lupus Erythematosus and Multiple
      Sclerosis

<130> U30520EP

<160> 7

<170> PatentIn version 3.5

<210> 1
<211> 133
<212> PRT
<213> Homo sapiens

<400> 1

Ala Pro Met Thr Gln Thr Thr Pro Leu Lys Thr Ser Trp Val Asn Cys
1               5                   10                  15

Ser Asn Met Ile Asp Glu Ile Ile Thr His Leu Lys Gln Pro Pro Leu
            20                  25                  30

Pro Leu Leu Asp Phe Asn Asn Leu Asn Gly Glu Asp Gln Asp Ile Leu
        35                  40                  45

Met Glu Asn Asn Leu Arg Arg Pro Asn Leu Glu Ala Phe Asn Arg Ala
    50                  55                  60

Val Lys Ser Leu Gln Asn Ala Ser Ala Ile Glu Ser Ile Leu Lys Asn
65                  70                  75                  80

Leu Leu Pro Cys Leu Pro Leu Ala Thr Ala Ala Pro Thr Arg His Pro
                85                  90                  95

Ile His Ile Lys Asp Gly Asp Trp Asn Glu Phe Arg Arg Lys Leu Thr
            100                 105                 110

Phe Tyr Leu Lys Thr Leu Glu Asn Ala Gln Ala Gln Gln Thr Thr Leu
        115                 120                 125

Ser Leu Ala Ile Phe
    130

<210> 2
<211> 4
<212> PRT
<213> Homo sapiens

<400> 2

Ser Trp Val Asn
1

<210> 3
<211> 22
<212> PRT
<213> Homo sapiens

<400> 3

Gln Pro Pro Leu Pro Leu Leu Asp Phe Asn Asn Leu Asn Gly Glu Asp
1               5                   10                  15

Gln Asp Ile Leu Met Glu
            20

<210> 4
<211> 18
<212> PRT
<213> Homo sapiens

<400> 4

Ala Pro Met Thr Gln Thr Thr Pro Leu Lys Thr Ser Trp Val Asn Cys
1               5                   10                  15

Ser Asn

<210> 5
<211> 22
<212> PRT
<213> Homo sapiens

<400> 5

Thr Phe Tyr Leu Lys Thr Leu Glu Asn Ala Gln Ala Gln Gln Thr Thr
1               5                   10                  15

Leu Ser Leu Ala Ile Phe
            20

<210> 6
<211> 133
<212> PRT
<213> Homo sapiens

<400> 6

Ala Pro Met Thr Gln Thr Thr Ser Leu Lys Thr Ser Trp Val Asn Cys
1               5                   10                  15

Ser Asn Met Ile Asp Glu Ile Ile Thr His Leu Lys Gln Pro Pro Leu
            20                  25                  30

Pro Leu Leu Asp Phe Asn Asn Leu Asn Gly Glu Asp Gln Asp Ile Leu
        35                  40                  45

Met Glu Asn Asn Leu Arg Arg Pro Asn Leu Glu Ala Phe Asn Arg Ala
    50                  55                  60

Val Lys Ser Leu Gln Asn Ala Ser Ala Ile Glu Ser Ile Leu Lys Asn
65                  70                  75                  80

Leu Leu Pro Cys Leu Pro Leu Ala Thr Ala Ala Pro Thr Arg His Pro
                85                  90                  95

Ile His Ile Lys Asp Gly Asp Trp Asn Glu Phe Arg Arg Lys Leu Thr
            100                 105                 110

Phe Tyr Leu Lys Thr Leu Glu Asn Ala Gln Ala Gln Gln Thr Thr Leu
        115                 120                 125

Ser Leu Ala Ile Phe
    130

<210>  7
<211>  18
<212>  PRT
<213>  Homo sapiens

<400>  7

Ala Pro Met Thr Gln Thr Thr Ser Leu Lys Thr Ser Trp Val Asn Cys
1               5                   10                  15

Ser Asn

## Description

The present invention relates to anti-IL-3 antibodies or IL-3 binding fragments thereof for use in the treatment of an autoimmune disease selected from the group consisting of systemic lupus erythematosus and multiple sclerosis, and to pharmaceutical compositions comprising such an antibody or antibody fragment.

Autoimmune diseases arise when the immune system inappropriately attacks substances or tissues that are normally present in the body. A large number of autoimmune diseases are known. Examples include diabetes mellitus type 1 (insulin-dependent diabetes mellitus), multiple sclerosis, Sjögren's syndrome, rheumatoid arthritis, Addison's Disease, Hashimoto's thyroiditis, Graves' disease, systemic lupus erythematosus (SLE), and allergies.

Of these, systemic lupus erythematosus (SLE) is a systemic autoimmune disease (or autoimmune connective tissue disease) that can affect any part of the body. The immune system's attack on the body's cells and tissue results in inflammation and tissue damage. The clinical course of SLE is variable and in most cases characterized by periods of remissions and relapses. SLE most often harms the heart, joints, skin, lungs, blood vessels, liver, kidneys, hematological system and nervous system. SLE affects mainly females with a female: male ratio of 10:1. The prevalence of SLE is 200-1500 / 1 million and the incidence is 10-250 / 1 million / year. Typical age of onset is 16-55 years (65% of cases). Although several genetic factors (e.g. HLA-DR2/3, MFG-E8, IRF4, IRAK1, Stat4, OX40L, PD1, FcgRII, Clq, C4) have been described, the cause of SLE is largely unknown. About 75 % of SLE patients develop renal disease and about 75 % of patients with renal disease have the more severe type WHO class III / IV. SLE can be diagnosed e.g. using the criteria developed by the American College of Rheumatology (Arthritis Rheum. 1997;40(9):1725). There is no cure for SLE available at this time.

Multiple sclerosis (MS) is an autoimmune disease in which the insulating covers of nerve cells in the brain and spinal cord are damaged. This damage disrupts the ability of parts of the nervous system to communicate, resulting in a wide range of signs and symptoms, including physical, mental, and sometimes psychiatric problems. The underlying mechanism is thought to involve destruction of the myelin-producing cells by the immune system. MS primarily affects women of child-bearing age and Northern European descent. MS is characterized by multifocal areas of demyelination with loss of oligodendrocytes and astroglial scarring. Axonal injury is also a prominent feature of MS. Multiple sclerosis is a clinical diagnosis. Certain criteria (e.g. the McDonald criteria) were developed for diagnosis (Ann Neurol. 2011;69(2):292). Clinical findings alone or in combination with imaging (MRI) are used to demonstrate a dissemination of central nervous system (CNS) lesions in both space and time. Although several environmental and genetic factors have been found to be associated with MS, the cause of MS is largely unknown. At present, there is no known cure for multiple sclerosis.

In the absence of an effective specific treatment option, SLE and MS are mostly treated by general immunosuppression. SLE affecting organs like the kidney (WHO III/IV), lung or CNS is mainly treated with steroids, cyclophosphamide, mycophenolate, azathioprin, and other immuosuppressants. Typically less than 60% of the SLE patients with organ involvement (especially of the kidney) respond to currently available treatments. In addition they often show relapses after reduction of immunosuppression.

Treatment of MS depends on the disease pattern. Relapsing-remitting multiple sclerosis (RRMS) is treated with steroids (for acute attacks) and with immunomodulatory agents ( e.g. interferons, glatiramer acetate, natalizumab, alemtuzumab, fingolimod, teriflunomide, cyclophosphamide, daclizumab) to decrease the relapse rates and to slower accumulation of brain lesions. Treatment of the progressive forms of MS (e.g. secondary progressive MS (SPMS), primary progressive MS (PPMS)) is insufficient and includes steroids, cyclophosphamide, methotrexate and mitoxantrone.

While such treatment options used to treat SLE and MS are only modestly effective, they often have severe side effects, such as infections and development of tumors.

Thus, there is a need in the art for alternative treatment options for both SLE and MS. In particular, there is a need in the art for improved treatment options for SLE and MS. Moreover, there is a need in the art for treatment options for SLE and MS that have fewer and/or less severe side effects.

It is therefore an object of the present invention to provide for alternative treatment options for both SLE and/or MS. Moreover, it is an object of the present invention to provide for improved treatment options for SLE and/or MS. Furthermore, it is an object of the present invention to provide for treatment options for SLE and MS that have fewer and/or less severe side effects. Moreover, it is an object of the present invention to provide for treatment options for SLE and MS that are less immunosuppressing than treatment options known from the prior art. Furthermore, it is an object of the present invention to provide for treatment options for SLE and MS that give rise to fewer infections during treatment than treatment options known from the prior art. Moreover, it is an object of the present invention to provide for treatment options for SLE and MS that have a reduced risk of inducing tumors compared to treatment options known from the prior art. Furthermore, it is an object of the present invention to provide for treatment options for SLE and MS that can readily be combined with other treatment options. Moreover, it is an object of the present invention to provide for treatment options for SLE to which a higher percentage of SLE patients with organ involvement (especially of the kidney) respond than to treatments known from the prior art. Moreover, it is an object of the present invention to provide for treatment options for SLE that show a reduced rate of relapses after reduction of immunosuppression compared to treatment options known from the prior art. Moreover, it is an object of the present invention to provide for treatment options for progressive forms of MS (e.g. secondary progressive MS (SPMS), primary progressive MS (PPMS)) and for decrease of relapse rates in remitting relapsing forms of MS, that are more effective compared to treatment options known from the prior art. The objects of the present invention are solved by an anti-IL-3 antibody or an IL-3 binding fragment thereof for use in the treatment of an autoimmune disease.

The following embodiments are, where applicable, embodiments of any of the solutions to the objects of the present invention. Moreover, the following embodiments can, wherever this does not lead to logical contradictions, be combined without restrictions. Thus, the present disclosure shall encompass, even if not explicitly spelled out in the following, any feasible combination of the following embodiments.

In one embodiment, said autoimmune disease is characterized by an increased plasma IL-3 level compared to a healthy state.

In one embodiment, said autoimmune disease is selected from the group consisting of systemic lupus erythematosus and multiple sclerosis. Preferably, said systemic lupus erythematosus is characterized by an increased plasma IL-3 level compared to a healthy state.

Preferably, said multiple sclerosis is characterized by an increased plasma IL-3 level compared to a healthy state.

In one embodiment, said autoimmune disease is systemic lupus erythematosus. Preferably, said systemic lupus erythematosus is characterized by an increased level of IL-3 expression compared to a healthy state. In one embodiment, said autoimmune disease is multiple sclerosis. Preferably, said multiple sclerosis is characterized by an increased level of IL-3 expression compared to a healthy state. Preferably, said multiple sclerosis is relapsing-remitting multiple sclerosis (RRMS), primary progressive multiple sclerosis (PPMS), secondary progressive multiple sclerosis (SPMS) or progressive relapsing multiple sclerosis (PRMS). Preferably, said multiple sclerosis is an acute attack in a relapsing-remitting multiple sclerosis (RRMS). In one embodiment, said autoimmune disease is lupus nephritis. Preferably, said lupus nephritis is characterized by an increased level of IL-3 expression compared to a healthy state.

In one embodiment, said anti-IL-3 antibody or said IL-3 binding fragment is administered to a patient in need thereof. Preferably, said patient is a mammal, more preferably said patient is a mouse, rat or human being, more preferably said patient is a human being.

In one embodiment, said anti-IL-3 antibody or said IL-3 binding fragment thereof is for use in a patient who has an increased plasma level of IL-3 compared to a healthy individual. Preferably, said plasma level of IL-3 is determined by enzyme-linked immunosorbent assay (ELISA).

In one embodiment, said anti-IL-3 antibody is a monoclonal, polyclonal or chimeric antibody, or a combination thereof. In one embodiment, said IL-3 binding fragment of said anti-IL-3 antibody is a fragment of a monoclonal, polyclonal or chimeric antibody, or a combination thereof.

In one embodiment, said anti-IL-3 antibody or said IL-3 binding fragment thereof is not immunogenic in a human subject.

In one embodiment, said anti-IL-3 antibody is a humanized antibody. In one embodiment, said anti-IL-3 antibody is a human antibody. In one embodiment, said anti-IL-3 antibody is a synthetic antibody.

In one embodiment, said anti-IL-3 antibody is of antibody isotype IgG, IgA, IgM, IgD, or IgE. Preferably, said anti-IL-3 antibody is of antibody isotype IgG.

In one embodiment, said anti-IL-3 antibody is obtained by a method comprising the step of immunizing an animal with a protein or peptide comprising or consisting of the amino acid sequence of SEQ ID NO: 1 or an immunogenic fragment thereof, or a nucleic acid or host cell expressing said protein or peptide or immunogenic fragment thereof, or comprising or consisting of the amino acid sequence of SEQ ID NO: 6 or an immunogenic fragment thereof, or a nucleic acid or host cell expressing said protein or peptide or immunogenic fragment thereof. Preferably, said anti-IL-3 antibody is obtained by a method comprising the step of immunizing an animal with a fragment of human IL-3 comprising or consisting of residues 17-133, preferably residues 21-133 of the human IL-3 amino acid sequence according to SEQ ID NO: 1 or SEQ ID NO: 6, preferably SEQ ID NO: 1. Preferably, said anti-IL-3 antibody is obtained by a method comprising the step of immunizing an animal with a fragment of human IL-3 comprising or consisting of
a) residues 12-15 of the human IL-3 amino acid sequence (SEQ ID NO: 2),
b) residues 29-50 of the human IL-3 amino acid sequence (SEQ ID NO: 3),
c) the 18 most N-terminal amino acids of the human IL-3 amino acid sequence (SEQ ID NO: 4 or SEQ ID NO: 7, preferably SEQ ID NO: 4), or
d) the 22 most C-terminal amino acids of the human IL-3 amino acid sequence (SEQ ID NO: 5),
more preferably b) or d).

Preferably, said fragment of human IL-3 has a length of at least 100, more preferably at least 80, more preferably at least 50, more preferably at least 40, more preferably at least 30, more preferably at least 22, more preferably at least 20, more preferably at least 10 amino acids. Most preferably, said fragment of human IL-3 has a length of at least 10 amino acids. Preferably, said fragment of human IL-3 has a length of up to 100, more preferably up to 80, more preferably up to 50, more preferably up to 40, more preferably up to 30, more preferably up to 22, more preferably up to 20, more preferably up to 10 amino acids. Most preferably, said fragment of human IL-3 has a length of up to 22 amino acids.

In one embodiment, said anti-IL-3 antibody is obtained by a method comprising the step of immunizing a rabbit, rat, mouse, chicken, goat, guinea pig, hamster, horse, or sheep. In one embodiment, said anti-IL-3 antibody is obtained by a method comprising the step of immunizing a rabbit. In one embodiment, said anti-IL-3 antibody is obtained by a method comprising the step of immunizing a mouse.

In one embodiment, said anti-IL-3 antibody or said IL-3 binding fragment thereof binds to the human IL-3 protein. In one embodiment, said anti-IL-3 antibody or said IL-3 binding fragment thereof is specific for the human IL-3 protein. In one embodiment, said anti-IL-3 antibody or said IL-3 binding fragment thereof does not bind to IL-3 proteins of other species besides human.

In one embodiment, said anti-IL-3 antibody or said IL-3 binding fragment thereof binds to IL-3, preferably to human IL-3, with an affinity (K_{D}) of at least 10⁻⁵ M, preferably at least 10⁻⁶ M, more preferably at least 10⁻⁷ M, more preferably at least 10⁻⁸ M, more preferably at least 10⁻⁹ M. In a preferred embodiment, said anti-IL-3 antibody or said IL-3 binding fragment thereof binds to IL-3, preferably to human IL-3, with an affinity (K_{D}) of at least 10⁻⁷ M.

In one embodiment, said anti-IL-3 antibody or said IL-3 binding fragment thereof is capable of preventing IL-3 from binding to and/or activating its receptor upon binding of said anti-IL-3 antibody or said IL-3 binding fragment thereof to IL-3. In one embodiment, binding of said anti-IL-3 antibody or said IL-3 binding fragment thereof to IL-3 prevents IL-3 from binding to and/or activating its receptor. Preferably, said receptor is the interleukin-3 receptor. Preferably, said binding or lack of binding of IL-3 to its receptor is determined by enzyme-linked immunosorbent assay (ELISA), more preferably by enzyme-linked immunosorbent assay (ELISA) using recombinant IL-3 receptor alpha-chain, or by determining binding of labelled IL-3, preferably IL-3 labelled with a fluorescent or radioactive label, to IL-3 receptor expressing cells, preferably by immunofluorescence or immunostaining/flow cytometric analysis of cells expressing the IL-3 receptor. Preferably, said cells expressing the IL-3 receptor are selected from the group consisting of basophils, plasmacytoid dendritic cells, monocytes and a (transfected or untransfected) cell line expressing the IL-3 receptor. Preferably, said binding or lack of binding of IL-3 to its receptor is determined by measurement of affinity (K_{D}) by surface plasmon resonance measurements. Preferably, said binding or lack of binding of IL-3 to its receptor is determined by measurement of affinity (K_{D}) by flow cytometry, cell based ELISA or detection of bound radioactivity. Preferably, said ability or failure of IL-3 to activate its receptor is determined by measuring cellular responses of IL-3 receptor positive cells, more preferably by determining IL-3 induced proliferation of TF-1 cells or IL-3 induced activation of basophils.

In one embodiment, said anti-IL-3 antibody or said IL-3 binding fragment thereof is capable of decreasing the plasma level of unbound IL-3 in said patient upon administration of said antibody or fragment thereof to said patient.

In one embodiment, said anti-IL-3 antibody or said IL-3 binding fragment thereof binds to a portion of the IL-3 amino acid sequence which portion consists of
a) residues 12-15 of the human IL-3 amino acid sequence (SEQ ID NO: 2),
b) residues 29-50 of the human IL-3 amino acid sequence (SEQ ID NO: 3),
c) the 18 most N-terminal amino acids of the human IL-3 amino acid sequence (SEQ ID NO: 4 or SEQ ID NO: 7, preferably SEQ ID NO: 4), or
d) the 22 most C-terminal amino acids of the human IL-3 amino acid sequence (SEQ ID NO: 5),
preferably b) or d).

In one embodiment, said anti-IL-3 antibody or said IL-3 binding fragment thereof binds to a portion of the IL-3 amino acid which portion consists of residues 17-133, preferably residues 21-133 of the human IL-3 amino acid sequence according to SEQ ID NO: 1 or SEQ ID NO: 6, preferably SEQ ID NO: 1.

In one embodiment, said anti-IL-3 antibody or said IL-3 binding fragment thereof does not bind to a portion of the IL-3 amino acid which portion consists of residues 1-16, preferably residues 1-20 of the human IL-3 amino acid sequence according to SEQ ID NO: 1 or SEQ ID NO: 6.

In one embodiment, said anti-IL-3 antibody or said IL-3 binding fragment thereof binds to an epitope within human IL-3 which epitope is capable of binding to an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F 15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems and
- monoclonal anti-IL-3 antibody Clone 4806 from R&D Systems,
preferably to an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F 15-216;
- monoclonal anti-IL-3 antibody Clone 4815 from R&D Systems and
- monoclonal anti-IL-3 antibody Clone 4806 from R&D Systems.
Preferably, said binding is specific binding.

In one embodiment, said anti-IL-3 antibody or said IL-3 binding fragment thereof competes with an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably with an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems
in a competitive binding assay.

In one embodiment, said anti-IL-3 antibody or said IL-3 binding fragment thereof binds to an epitope within human IL-3 comprising or comprising a portion of the sequence defined by
a) residues 12-15 of the human IL-3 amino acid sequence (SEQ ID NO: 2),
b) residues 29-50 of the human IL-3 amino acid sequence (SEQ ID NO: 3),
c) the 18 most N-terminal amino acids of the human IL-3 amino acid sequence (SEQ ID NO: 4 or SEQ ID NO: 7, preferably SEQ ID NO: 4), or
d) the 22 most C-terminal amino acids of the human IL-3 amino acid sequence (SEQ ID NO: 5)
preferably b) or d).

In one embodiment, said anti-IL-3 antibody or said IL-3 binding fragment thereof binds to an epitope within the human IL-3 sequence which does not overlap with the 16, preferably 20, most N-terminal amino acids of the human IL-3 amino acid sequence according to SEQ ID NO: 1 or SEQ ID NO: 6, preferably SEQ ID NO: 1.

In one embodiment, said anti-IL-3 antibody or said IL-3 binding fragment thereof binds to an epitope which lies within residues 17-133, preferably residues 21-133, of the human IL-3 amino acid sequence according to SEQ ID NO: 1 or SEQ ID NO: 6, preferably SEQ ID NO: 1.

In one embodiment, said anti-IL-3 antibody or said IL-3 binding fragment thereof binds to an epitope within human IL-3 which epitope comprises one or several of the amino acids S 17, N18, D21, E22, T25, E43, M49, R94, P96, R108, F113, and E119 of the human IL-3 amino acid sequence shown in SEQ ID NO: 1 or SEQ ID NO: 6, preferably in SEQ ID NO: 1.

In one embodiment, said anti-IL-3 antibody is selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F 15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems,
and/or said IL-3 binding fragment thereof is an IL-3 binding fragment of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F 15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said anti-IL-3 antibody comprises an amino acid sequence at least 90%, preferably at least 95%, more preferably at least 98%, more preferably at least 99% identical to the amino acid sequence of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F 14-746;
- monoclonal anti-IL-3 antibody F 13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said IL-3 binding fragment of said anti-IL-3 antibody comprises a fragment of an amino acid sequence at least 90%, preferably at least 95%, more preferably at least 98%, more preferably at least 99% identical to the amino acid sequence of an antibody selected from the group consisting of
- monoclonal anti-IL-3 antibody F14-570;
- monoclonal anti-IL-3 antibody F14-746;
- monoclonal anti-IL-3 antibody F 13-267;
- monoclonal anti-IL-3 antibody F 15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said IL-3 binding fragment of said anti-IL-3 antibody binds to IL-3 through a portion of the sequence of said IL-3 binding fragment that has the same sequence as a portion of the amino acid sequence of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F 15-216;
- monoclonal anti-IL-3 antibody Clone 4815 from R&D Systems; and
- monoclonal anti-IL-3 antibody Clone 4806 from R&D Systems,
or through a portion of the sequence of said IL-3 binding fragment that has an amino acid sequence at least 90%, preferably at least 95%, more preferably at least 98%, more preferably at least 99% identical to a portion of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said anti-IL-3 antibody consists of the amino acid sequence of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems,
or of an amino acid sequence at least 90%, preferably at least 95%, more preferably at least 98%, more preferably at least 99% identical to the amino acid sequence of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said IL-3 binding fragment of said anti-IL-3 antibody is a fragment of the amino acid sequence of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F 15-216;
- monoclonal anti-IL-3 antibody Clone 4815 from R&D Systems; and
- monoclonal anti-IL-3 antibody Clone 4806 from R&D Systems,
or is a fragment of an amino acid sequence at least 90%, preferably at least 95%, more preferably at least 98%, more preferably at least 99% identical to the amino acid sequence of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said anti-IL-3 antibody or said IL-3 binding fragment thereof comprises an amino acid sequence that is identical to the amino acid sequence of the V_{H} region of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems,
or that has an amino acid sequence at least 90%, preferably at least 95%, more preferably at least 98%, more preferably at least 99% identical to the amino acid sequence of the V_{H} region of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said anti-IL-3 antibody or said IL-3 binding fragment thereof comprises an amino acid sequence that is identical to the amino acid sequence of the V_{L} region of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems,
or that has an amino acid sequence at least 90%, preferably at least 95%, more preferably at least 98%, more preferably at least 99% identical to the amino acid sequence of the V_{L} region of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
preferably of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 4815 from R&D Systems; and
- monoclonal anti-IL-3 antibody Clone 4806 from R&D Systems.

In one embodiment, said anti-IL-3 antibody or said IL-3 binding fragment thereof is specific for IL-3. In one embodiment, said anti-IL-3 antibody or said IL-3 binding fragment thereof does not bind to interleukins other than IL-3.

In one embodiment, if said autoimmune disease is systemic lupus erythematosus, said anti-IL-3 antibody or said IL-3 binding fragment thereof is capable of reducing cellular infiltration in the kidney, reducing acute damage in the kidney, reducing chronic damage in the kidney, reducing immunoglobulin deposition in the kidney and reducing fibrosis in the kidney in said patient upon administration of said antibody or fragment thereof to said patient.

In one embodiment, if said autoimmune disease is multiple sclerosis, said anti-IL-3 antibody or said IL-3 binding fragment thereof is capable of reducing leukocyte infiltration in the brain, preferably during acute attacks, reducing demyelination and reducing axonal damage in said patient upon administration of said antibody or fragment thereof to said patient.

In one embodiment, said anti-IL-3 antibody or said IL-3 binding fragment thereof is administered by a route selected from the group consisting of intravenously, intramuscularly, subcutaneously, intraperitoneally, topically, orally, rectally, and inhalation administration. In one embodiment, said anti-IL-3 antibody or said IL-3 binding fragment thereof is administered intravenously, preferably by injection, or subcutaneously, preferably by injection.

In one embodiment, said anti-IL-3 antibody or said IL-3 binding fragment thereof is administered daily, preferably once every day. In one embodiment, said anti-IL-3 antibody or said IL-3 binding fragment thereof is administered once every week. In one embodiment, said anti-IL-3 antibody or said IL-3 binding fragment thereof is administered once every two weeks. In one embodiment, said anti-IL-3 antibody or said IL-3 binding fragment thereof is administered by bolus administration. In one embodiment, said anti-IL-3 antibody or said IL-3 binding fragment thereof is administered for up to 5 days, preferably up to 4 days, more preferably up to 3 days, more preferably up to 2 days, more preferably 1 day. In one embodiment, said anti-IL-3 antibody or said IL-3 binding fragment thereof is administered for at least one week, preferably at least two weeks, more preferably at least three weeks, more preferably at least 4 weeks, more preferably at least 8 weeks, more preferably at least 12 weeks.

In one embodiment, simultaneously with said administration of said anti-IL-3 antibody or IL-3 binding fragment thereof said patient is subjected to one or more additional treatments for treating systemic lupus erythematosus, wherein, preferably, said treatment consists of the administration of one or more immunosuppressants, preferably selected from the group consisting of steroids, cyclophosphamide, mycophenolate and azathioprin. In one embodiment, simultaneously with said administration of said anti-IL-3 antibody or IL-3 binding fragment thereof said patient is subjected to one or more additional treatments for treating multiple sclerosis, wherein, preferably, said treatment consists of the administration of one or more agents selected from the group consisting of steroids, cyclophosphamide, methotrexate, mitoxantrone and immunomodulatory agents preferably selected from the group consisting of interferons, glatiramer acetate, natalizumab, alemtuzumab, fingolimod, teriflunomide, cyclophosphamide and daclizumab.

The objects of the present invention are also solved by a pharmaceutical composition comprising at least one pharmaceutically acceptable carrier, diluent and/or excipient and an anti-IL-3 antibody or IL-3 binding fragment thereof as defined above for use in the treatment of an autoimmune disease.

In such pharmaceutical composition, said anti-IL-3 antibody and said IL-3 binding fragment thereof, and said autoimmune disease are as defined in the embodiments above.

In one embodiment, said pharmaceutical composition further comprises an agent effective for treatment of systemic lupus erythematosus, wherein, preferably, said agent is an immunosuppressant, preferably selected from the group consisting of steroids, cyclophosphamide, mycophenolate and azathioprin, for combined use for treating systemic lupus erythematosus. In one embodiment, said pharmaceutical composition further comprises an agent effective for treatment of multiple sclerosis, wherein, preferably, said agent is selected from the group consisting of steroids, cyclophosphamide, methotrexate, mitoxantrone and immunomodulatory agents preferably selected from the group consisting of interferons, glatiramer acetate, natalizumab, alemtuzumab, fingolimod, teriflunomide, cyclophosphamide and daclizumab for combined use for treating multiple sclerosis.

The objects of the present invention are also solved by a method of treatment of an autoimmune disease, said method comprising administration of an effective amount of an anti-IL-3 antibody or an IL-3 binding fragment thereof to a patient in need thereof.

In such method, said anti-IL-3 antibody and said IL-3 binding fragment thereof, said autoimmune disease and said administration are as defined in the embodiments above.

The objects of the present invention are also solved by the use of an anti-IL-3 antibody or an IL-3 binding fragment in the manufacture of a medicament for the treatment of an autoimmune disease.

In one embodiment, said treatment occurs by administration of said anti-IL-3 antibody or said IL-3 binding fragment to a patient in need thereof.

In such use and the embodiment referring to it, said anti-IL-3 antibody and said IL-3 binding fragment thereof, said autoimmune disease and said administration are as defined in the embodiments above.

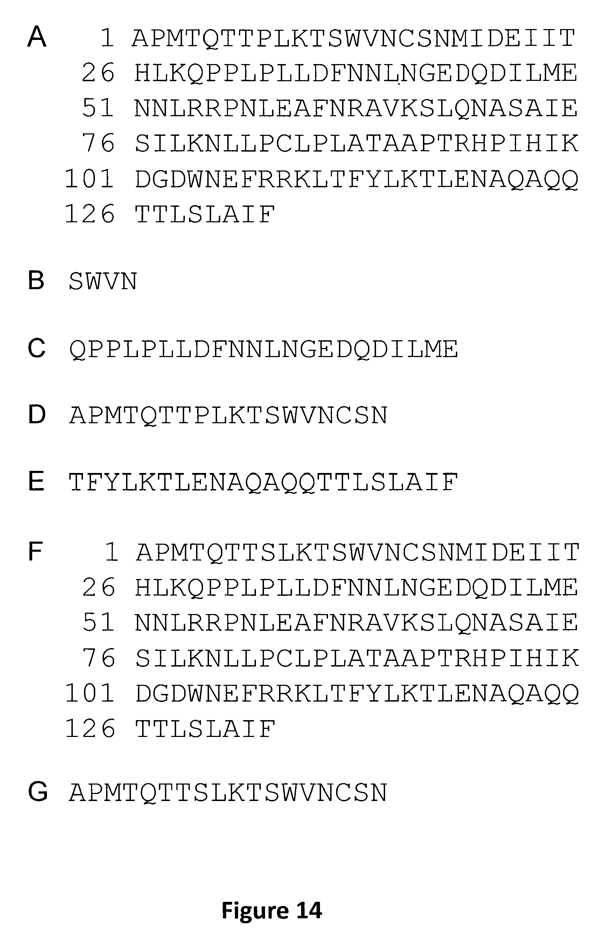

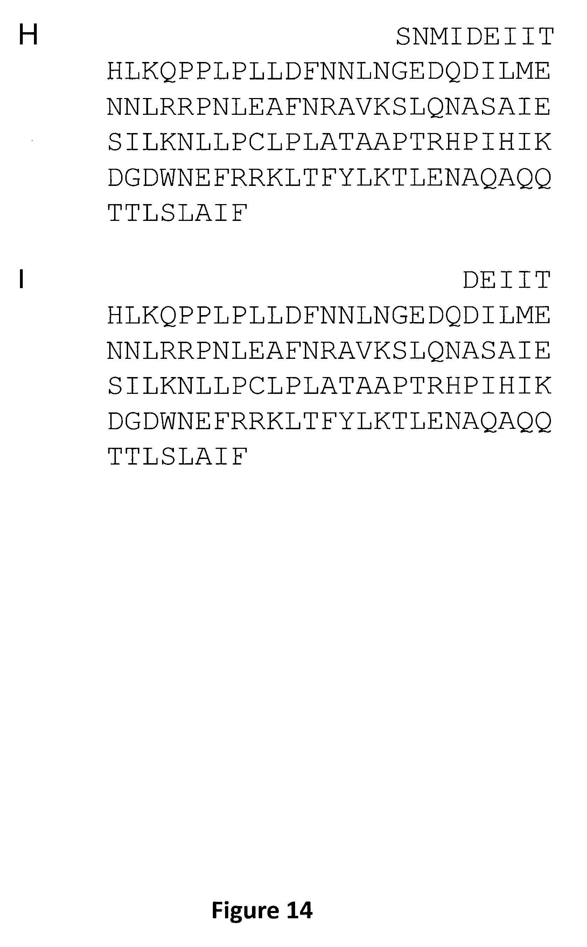

As used herein, the terms "IL-3" or "interleukin-3" are synonymous and refer to the naturally occurring, or endogenous mammalian interleukin-3 proteins and to proteins having an amino acid sequence which is the same as that of a naturally occurring or endogenous corresponding mammalian IL-3 protein (e.g. recombinant proteins, synthetic proteins (i.e., produced using the methods of synthetic organic chemistry)). Such proteins can for example be recovered or isolated from a source which naturally produces IL-3, or be produced by methods of recombinant protein expression. The terms "IL-3" or "interleukin-3" comprise the IL-3 proteins of different mammalian organisms, such as human, mouse, or rat IL-3. In one embodiment, the terms relate exclusively to human IL-3. Preferably, the amino acid sequence of human IL-3 is provided by SEQ ID NO: 1 or SEQ ID NO: 6 (see Fig. 14, SEQ ID NO: 6 refers to a known alternative allelic form with a polymorphism (at position 8 P is replaced with S)). More preferably, the amino acid sequence of human IL-3 is provided by SEQ ID NO: 1.
Anti-IL-3 antibodies specifically binding to human IL-3 are commercially available, for example from R&D System Clones 4806 and 4815 (Catalog No. MAB203 and No. MAB603), and from BD Biosciences Clones BVD3-1 F9 and BVD8-3G11 (Catalog No. 554674 Biotin Rat Anti-Human IL-3 0.5 mg; Catalog No. 554672 Purified Rat Anti-Human IL-3 0.5 mg) or have been published (F14-570, F14-746, J Immunol. 1991, 146:893-898). Other anti-IL-3 antibodies can be used as well.

The term " unbound IL-3", as used herein, refers to IL-3 protein molecules that are neither bound to a receptor nor bound to an antibody.

The term "antibody", as used herein, is used interchangeably with the term "immunoglobulin" and refers to a glycoprotein comprising at least two heavy (H) chains and two light (L) chains inter-connected by disulfide bonds. The term also includes all recombinant forms of antibodies, e.g. antibodies expressed in prokaryotes, unglycosylated antibodies and derivatives as described below. There are five different types of heavy chains, which define antibody isotypes of different functional activity: IgM, IgD, IgG, IgA and IgE. Each heavy chain comprises a heavy chain variable region (abbreviated herein as V_{H} region) and a heavy chain constant region. Each light chain comprises a light chain variable region (abbreviated herein as V_{L} region) and a light chain constant region. The V_{H} and V_{L} regions can be further subdivided into regions of hypervariability, termed complementarity determining regions (CDR), interspersed with regions that are more conserved, termed framework regions (FR). The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "monoclonal antibody", as used herein, refers to a preparation of antibody molecules of single molecular composition. A monoclonal antibody displays a single binding specificity and affinity for a particular epitope. In one embodiment, the monoclonal antibodies are produced by a hybridoma which includes a B cell obtained from a non-human animal, e.g. mouse, fused to an immortalized cell.

As used herein, the term "polyclonal antibody" refers to a heterogeneous pool of antibodies produced by a number of different B lymphocytes. Different antibodies in the pool recognize and specifically bind different epitopes.

The term "chimeric antibody" refers to those antibodies wherein one portion of each of the amino acid sequences of heavy and light chains is based upon a sequence in an antibody/sequences in antibodies derived from a particular species or belonging to a particular class, while the remaining segment of the chain is based upon a sequence/sequences in another. Typically the variable region of both light and heavy chains mimics the variable regions of antibodies derived from one species of mammals, while the constant portions are based upon sequences of antibodies derived from another. Preferably, the constant portions are based upon sequences of antibodies derived from human.

The term "humanized antibody", as used herein, refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "human antibody", as used herein, refers to an antibody which comprises only sequences derived from human immunoglobulin sequences.

The term "synthetic antibody", as used herein, relates to an antibody which is generated using recombinant DNA technology (such as by phage display) or DNA synthesis.

The term "anti-IL-3 antibody", as used herein, refers to an antibody which binds to the IL-3 protein. Preferably, said binding is specific binding. If the present application refers to an "IL-3 binding fragment" of an anti-IL-3 antibody, this relates to a fragment of that anti-IL-3 antibody which fragment is capable of binding to the IL-3 protein. Preferably, said binding is specific binding. The term "anti-IL-3 antibody" also includes monoclonal, polyclonal, humanized, human, and synthetic antibodies, single chain, bispecific, and simianized antibodies, as well as aptamers. In some embodiments, the term "anti-IL-3 antibody" does not include aptamers.

The present inventors have found that anti-IL-3 antibodies with epitopes that do not include the site of polymorphism within the human IL-3 sequence (residue 8 of the human IL-3 sequence) have the highest chance of binding to both polymorphic forms of human IL-3. Since epitopes are often 8, sometimes 10-12 amino acids in length, this means that an antibody generated against an IL-3 sequence lacking the 16, preferably 20, most N-terminal amino acids of the human IL-3 sequence (and thus an anti-IL-3 antibody binding to an epitope within the human IL-3 sequence which does not overlap with the 16, preferably 20, most N-terminal amino acids of the human IL-3 amino acid sequence, i.e. an anti-IL-3 antibody binding to an epitope which lies within residues 17-133, preferably residues 21-133, of the human IL-3 amino acid sequence) has the highest chance of binding to both polymorphic forms of human IL-3.

The term "aptamer", as used herein, refers to DNA or RNA molecules that have been selected from random pools based on their ability to bind other molecules. Aptamers have been selected which bind nucleic acid, proteins, small organic compounds, and even entire organisms. A database of aptamers is maintained at http //aptamer icmb utexas edu/. More specifically, aptamers can be classified as DNA or RNA aptamers or peptide aptamers. Whereas the former consist of (usually short) strands of oligonucleotides, the latter consist of a short variable peptide domain, attached at both ends to a protein scaffold. Nucleic acid aptamers are nucleic acid species that have been engineered through repeated rounds of *in vitro* selection or equivalently, SELEX (systematic evolution of ligands by exponential enrichment) to bind to various molecular targets such as small molecules proteins, nucleic acids, and even cells, tissues and organisms. Peptide aptamers are proteins that are designed to interfere with other protein interactions inside cells. They consist of a variable peptide loop attached at both ends to a protein scaffold. This double structural constraint greatly increases the binding affinity of the peptide aptamer (to nanomolar range). The variable loop length is typically 10 to 20 amino acids, and the scaffold may be any protein which has good solubility properties. Currently, the bacterial protein Thioredoxin-A is the most used scaffold protein, the variable loop being inserted within the reducing active site, which is a -Cys-Gly-Pro-Cys-loop in the wild protein, the two cysteins lateral chains being able to form a disulfide bridge. Peptide aptamer selection can be made using different systems, but the most used is currently the yeast two-hybrid system. Aptamers offer the utility for biotechnological and therapeutic applications as they offer molecular recognition properties that rival those of the commonly used biomolecules. In addition to their discriminate recognition, aptamers offer the advantage that they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, etc. are available to scientists with which the half-life of aptamers easily can be increased to the day or even week time scale.

Examples of IL-3 binding fragments of an anti-IL-3 antibody include separated light and heavy chains, Fab, Fab/c, Fv, Fab', and F(ab')2 fragments, including epitope-binding fragments of any of the antibodies and fragments mentioned above.

The term "MP2-8F8", as used herein, refers to a certain monoclonal antibody that was generated with recombinant mouse IL-3 as immunogen and is specific for mouse IL-3 (Abrams and Pearce, J Immunology (1988) 140, 131-137). MP2-8F8 can be obtained commercially for example from BD Biosciences (Catalog No. 554379 Purified NA/LE Rat Anti-Mouse IL-3 0.5 mg), R&D Systems (Catalog No. MAB403 Mouse IL-3 MAb (Clone MP28F8) 0.5 mg) or Biozol (BLD-503902 Purified anti-mouse IL-3, clone MP2-8F8, rat IgG 1 kappa 0.5 mg).

As used herein, the term "epitope" means a protein sequence / structure capable of binding to an antibody generated in response to such sequence, wherein the term "binding" herein preferably relates to specific binding.

The term "immunogenic", as used herein, refers to a peptide which, upon being administered to a subject, or taken up by the subject in other ways, elicits an immune response. Such immune response includes at least the generation of antibodies which specifically bind the immunogenic substance (i.e., a humoral response). An immunogenic substance may in addition elicit a cellular immunological response.

### Production of polyclonal antibodies:

Methods for production of polyclonal antibodies are well known to a person skilled in the art and can be found in Harlow & Lane, Antibodies: a Laboratory Manual (Cold Spring Harbor Laboratories, Cold Spring Harbor, NY: 1988). Polyclonal antibodies or antibodies may be produced by injecting a host animal such as rabbit, rat, goat, mouse or other animal with a suitable immunogen, e.g. full-length IL-3 protein or a fragment thereof (such as a carrier-conjugated peptide representing; common carriers are for example keyhole limpet hemocyanin (KLH) or bovine serum albumin (BSA)). The sera are extracted from the host animal and are screened to obtain polyclonal antibodies which are specific to the immunogen. Methods of screening for polyclonal antibodies are well known to those of ordinary skill in the art and include, for example, those disclosed in Harlow & Lane, Antibodies: a Laboratory Manual.

### Production of monoclonal antibodies:

Monoclonal antibodies of the invention can be produced by a variety of techniques, including conventional monoclonal antibody methodology, e.g., the standard somatic cell hybridization technique of Köhler and Milstein. Although somatic cell hybridization procedures are preferred, in principle, other techniques for producing monoclonal antibodies can be employed, e.g., viral or oncogenic transformation of B-lymphocytes or phage display techniques using libraries of antibody genes.

The preferred animal system for preparing hybridomas that secrete monoclonal antibodies is the murine system. Hybridoma production in the mouse is a very well established procedure. Immunization protocols and techniques for isolation of immunized splenocytes for fusion are known in the art. Fusion partners (e.g., murine myeloma cells) and fusion procedures are also known.

For details of recombinant antibody engineering see M. Welschof and J. Krauss, Recombinant antibodies for cancer therapy, ISBN-0-89603-918-8 and Benny K.C. Lo, Antibody Engineering, ISBN 1-58829-092-1.

### Immunizations for monoclonal antibody generation:

To generate monoclonal antibodies to IL-3, mice can be immunized with carrier-conjugated peptides derived from the IL-3 sequence, an enriched preparation of recombinantly expressed IL-3 antigen or fragments thereof and/or cells expressing IL-3. Alternatively, mice can be immunized with DNA encoding full length human IL-3 (e.g. SEQ ID NO: 1) or fragments thereof.

The immune response can be monitored over the course of the immunization protocol with plasma and serum samples being obtained by tail vein or retroorbital bleeds. Mice with sufficient titers of anti-IL-3 immunoglobulin can be used for fusions. Mice can be boosted intraperitoneally or intravenously with IL-3 three days before sacrifice and removal of the spleen to increase the rate of specific antibody secreting hybridomas.

### Generation of Hybridomas Producing Monoclonal Antibodies:

To generate hybridomas producing monoclonal antibodies to IL-3, splenocytes and lymph node cells from immunized mice can be isolated and fused to an appropriate immortalized cell line, such as a mouse myeloma cell line. The resulting hybridomas can then be screened for the production of antigen-specific antibodies. Individual wells can then be screened by ELISA for antibody secreting hybridomas. The antibody secreting hybridomas can be replated, screened again, and if still positive for anti-IL-3 monoclonal antibodies can be subcloned by limiting dilution. The stable subclones can then be cultured in vitro to generate antibody in tissue culture medium for characterization.

### Generation of Transfectomas Producing Monoclonal Antibodies:

Antibodies of the invention also can be produced in a host cell transfectoma using, for example, a combination of recombinant DNA techniques and gene transfection methods as are well known in the art.

For example, in one embodiment, the gene(s) of interest, e.g., antibody genes, can be ligated into an expression vector such as a eukaryotic expression plasmid such as used by the GS gene expression system disclosed in WO 87/04462 , WO 89/01036 and EP 338 841 or other expression systems well known in the art. The purified plasmid with the cloned antibody genes can be introduced in eukaryotic host cells such as CHO cells, NS/0 cells or HEK293 cells or alternatively other eukaryotic cells like plant derived cells, fungal or yeast cells. The method used to introduce these genes can be methods described in the art such as electroporation, lipofectine, lipofectamine or others. After introduction of these antibody genes in the host cells, cells expressing the antibody can be identified and selected. These cells represent the transfectomas which can then be amplified for their expression level and upscaled to produce antibodies. Recombinant antibodies can be isolated and purified from these culture supernatants and/or cells.

Alternatively, the cloned antibody genes can be expressed in other expression systems, including prokaryotic cells, such as microorganisms, e.g. *E*. *coli.*

Use of Partial Antibody Sequences to Express Intact Antibodies (i.e. humanization and chimerisation).

### a) Chimerization

Nonlabelled murine antibodies are highly immunogenic in man when repetitively applied leading to reduction of the therapeutic effect. The main immunogenicity is mediated by the heavy chain constant regions. The immunogenicity of murine antibodies in man can be reduced or completely avoided if respective antibodies are chimerized or humanized. Chimerisation of antibodies is achieved by joining of the variable regions of the murine antibody heavy and light chain with the constant region of human heavy and light chain (e.g. as described by Krauss et al., in Methods in Molecular Biology series, Recombinant antibodies for cancer therapy, ISBN-0-89603-918-8). In a preferred embodiment chimeric antibodies are generated by joining human kappa-light chain constant region to murine light chain variable region. In an also preferred embodiment chimeric antibodies can be generated by joining human lambda-light chain constant region to murine light chain variable region. The preferred heavy chain constant regions for generation of chimeric antibodies are IgG1, IgG3 and IgG4. Other preferred heavy chain constant regions for generation of chimeric antibodies are IgG2, IgA, IgD and IgM.

### b) Humanization

Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al. (1998) Nature 332: 323-327). Such framework sequences can be obtained from public DNA databases that include germline antibody gene sequences. These germline sequences will differ from mature antibody gene sequences because they will not include completely assembled variable genes, which are formed by V(D)J joining during B cell maturation. Germline gene sequences will also differ from the sequences of a high affinity secondary repertoire antibody at individual evenly across the variable region. For example, somatic mutations are relatively infrequent in the amino terminal portion of framework region 1 and in the carboxy-terminal portion of framework region 4. Furthermore, many somatic mutations do not significantly alter the binding properties of the antibody. For this reason, it is not necessary to obtain the entire DNA sequence of a particular antibody in order to recreate an intact recombinant antibody having binding properties similar to those of the original antibody (see WO 99/45962 ). Partial heavy and light chain sequences spanning the CDR regions are typically sufficient for this purpose. The partial sequence is used to determine which germline variable and joining gene segments contributed to the recombined antibody variable genes. The germline sequence is then used to fill in missing portions of the variable regions. Heavy and light chain leader sequences are cleaved during protein maturation and do not contribute to the properties of the final antibody. To add missing sequences, cloned cDNA sequences can be combined with synthetic oligonucleotides by ligation or PCR amplification. Alternatively, the entire variable region can be synthesized as a set of short, overlapping, oligonucleotides and combined by PCR amplification to create an entirely synthetic variable region clone. This process has certain advantages such as elimination or inclusion or particular restriction sites, or optimization of particular codons.

The nucleotide sequences of heavy and light chain transcripts from hybridomas are used to design an overlapping set of synthetic oligonucleotides to create synthetic V sequences with identical amino acid coding capacities as the natural sequences.

For both the heavy and light chain variable regions, the optimized coding and corresponding non-coding, strand sequences are broken down into 30-50 nucleotides approximately at the midpoint of the corresponding non-coding oligonucleotide. Thus, for each chain, the oligonucleotides can be assembled into overlapping double stranded sets that span segments of 150-400 nucleotides. The pools are then used as templates to produce PCR amplification products of 150-400 nucleotides. Typically, a single variable region oligonucleotide set will be broken down into two pools which are separately amplified to generate two overlapping PCR products. These overlapping products are then combined by PCR amplification to form the complete variable region. It may also be desirable to include an overlapping fragment of the heavy or light chain constant region in the PCR amplification to generate fragments that can easily be cloned into the expression vector constructs.

The reconstructed chimerized or humanized heavy and light chain variable regions are then combined with cloned promoter, leader, translation initiation, constant region, 3' untranslated, polyadenylation, and transcription termination sequences to form expression vector constructs. The heavy and light chain expression constructs can be combined into a single vector, co-transfected, serially transfected, or separately transfected into host cells which are then fused to form a host cell expressing both chains. Plasmids for use in construction of expression vectors for human IgG are described below. The plasmids were constructed so that PCR amplified V heavy and V kappa light chain cDNA sequences could be used to reconstruct complete heavy and light chain minigenes. These plasmids can be used to express completely human, or chimeric IgG1, Kappa or IgG4, Kappa antibodies. Similar plasmids can be constructed for expression of other heavy chain isotypes, or for expression of antibodies comprising lambda light chains.

Thus, in another aspect of the invention, the structural features of the anti-IL-3 antibodies of the invention, are used to create structurally related humanized anti-IL-3 antibodies that retain at least one functional property of the antibodies of the invention, such as binding to IL-3. More specifically, one or more CDR regions of mouse monoclonal antibodies can be combined recombinantly with known human framework regions and CDRs to create additional, recombinantly-engineered, humanized anti-IL-3 antibodies of the invention.

### Verification of (polyclonal or monoclonal) antibody binding to IL-3:

The ability of the antibody to bind IL-3 can be determined using standard binding assays, such as ELISA, Western Blot, or measurement of affinity (K_{D}) by surface plasmon resonance measurements (e.g. with a Biacore™ device, GE Healthcare Life Sciences, Piscataway, NJ).

### Purification of monoclonal anti-IL-3 antibodies:

To purify anti-IL-3 antibodies, selected hybridomas can be grown in two-liter spinner-flasks for monoclonal antibody purification. Alternatively, anti-IL-3 antibodies can be produced in dialysis based bioreactors. Supernatants can be filtered and, if necessary, concentrated before affinity chromatography with protein G-sepharose or protein A-sepharose. Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80 °C.

### Verification of antibody binding to a certain epitope

To determine whether an anti-IL-3 antibody binds to a certain epitope within the IL-3 sequence, mutations of amino acids are introduced into the IL-3 protein sequence at the site of the epitope, for example by site-directed mutagenesis. Subsequently, a binding assay (for example an ELISA) is used to test if the antibody still binds to the IL-3 protein with the mutated epitope.

Alternatively, the epitope of the antibody can be mapped by array-based oligo-peptide scanning. This technique uses a library of oligo-peptide sequences from overlapping and nonoverlapping segments of the target protein (here IL-3). Oligo-peptide sequences are synthetically prepared by known oligopeptide synthesis techniques. Subsequently, tests for their ability to bind the antibody of interest are carried out by methods known to the person of skill in the art, preferably by ELISA.

At some instances, the present application refers to a situation where a first antibody "competes" with second antibody "in a competitive binding assay". For example, the application may state that an "anti-IL-3 antibody competes with the monoclonal antibody X in a competitive binding assay". Such competitive binding is preferably determined by an enzyme-linked immunosorbent assay (ELISA) assay according to procedures known in the art: One of the antibodies is labelled (for example biotinylated) by standard techniques. The unlabelled antibody is immobilized, full-length IL-3 is captured by the immobilized antibody, and the second (labelled) antibody is tested in an ELISA assay for its ability to bind to the captured IL-3.

Alternatively, measurements with a surface plasmon resonance (SPR) device (e.g. Biacore™, GE Healthcare Life Sciences, Piscataway, NJ) can be carried out by immobilizing one antibody and measuring the binding of a complex of IL-3 with the second antibody or measuring the sequential binding of IL-3 and the second antibody.

The terms "binds" and "binding", as used herein, preferably relate to specific binding. In some embodiments, the terms "binds" is to be understood as "is capable of binding", and "binding" is to be understood as "capable of binding"; accordingly, the term "is specific for" is to be understood as "is capable of specifically binding (to)", the term "specifically binds" is to be understood as "is capable of specifically binding (to)", and "specific binding" is to be understood as "capability of specific binding (to)".

The terms "is specific for", "specifically binds" and "specific binding", as used for example in the context of a molecule A being specific for a molecule B or a molecule A specifically binding to a molecule B or a molecule A showing specific binding for a molecule B, refer to a situation where molecule A binds to molecule B, but does not bind to other unrelated molecules, or with substantially reduced affinities. Such binding can be measured by routine methods, for example by competition ELISA or by measurement of affinity (K_{D}) by surface plasmon resonance measurements (e.g. with a Biacore™ device, GE Healthcare Life Sciences, Piscataway, NJ). Similarly, a molecule A being specific for an epitope C or a molecule A specifically binding to epitope C or a molecule A showing specific binding for epitope C, refer to a situation where molecule A binds to epitope C, but does not bind to other unrelated epitopes, or with substantially reduced affinities.

The term "affinity (K_{D})" as used herein, refers to the dissociation equilibrium constant of a particular molecular interaction.

At some occasions, the present application refers to a situation where binding of a molecule A to IL-3 "prevents IL-3 from binding to its receptor". Whether binding of a molecule A to IL-3 prevents IL-3 from binding to its receptor can be determined by methods well-known to the skilled person, for example by immunofluorescence or immunostaining/flow cytometric analysis of cells expressing the IL-3 receptor, such as basophils, plasmacytoid dendritic cells, monocytes or a transfected or untransfected cell line expressing the IL-3 receptor, (for example by pre-incubation of IL-3 in the presence or absence of molecule A, incubation of cells expressing the IL-3 receptor with the pre-incubated IL-3, staining of the cells with an anti-IL-3 antibody to detect IL-3 bound to the receptor at the cell surface, control that the anti-IL-3 antibody can recognize IL-3 even if it is bound by molecule A), or by measurement of affinity (K_{D}) by surface plasmon resonance measurements.

For several antibodies it is known that their binding to human IL-3 prevents IL-3 from binding to its receptor, for example monoclonal anti-IL-3 antibodies F 14-570, F 14-746, F13-267 and F15-216 as published in J Immunol. 1991 Feb 1;146(3):893-8; monoclonal anti-IL-3 antibody Clone 11.14.6 and monoclonal anti-IL-3 antibody Clone 13.4.4 as described in European Patent Application No. EP 12169799.9 (filing date: May 29, 2012) and international patent application PCT/EP2013/061121 (accessible through DSMZ depositions DSMACC3163 and DSMACC3164, respectively); monoclonal anti-IL-3 antibody Clone 4815 from R&D Systems (Catalog Number MAB603) and monoclonal anti-IL-3 antibody Clone 4806 from R&D Systems (Catalog Number MAB203) (see also antibody datasheets at http://www.rndsystems.com/pdf/mab603.pdf and http://www.rndsystems.com/pdf/ mab203.pdf).

Moreover, it is known that the binding to human IL-3 of antibodies binding to certain epitopes within human IL-3 prevents IL-3 from binding to its receptor, for example antibodies binding to the region of
a) residues 12-15 of the human IL-3 amino acid sequence (see European Patent Application No. EP12169799.9 (filing date: May 29, 2012) and international patent application PCT/EP2013/061121),
b) residues 29-50 of the human IL-3 amino acid sequence (see J Immunol. 1991 Feb 1;146(3):893-8),
c) the 18 most N-terminal amino acids of the human IL-3 amino acid sequence (see EMBO J. 1991 Aug;10(8):2125-31), or
d) the 22 most C-terminal amino acids of the human IL-3 amino acid sequence (see EMBO J. 1991 Aug;10(8):2125-31).

The amino acid sequence of residues 12-15 of the human IL-3 amino acid sequence is provided by SEQ ID NO: 2.

The amino acid sequence of residues 29-50 of the human IL-3 amino acid sequence is provided by SEQ ID NO: 3.

The amino acid sequence of the 18 most N-terminal amino acids of the human IL-3 amino acid sequence is provided by SEQ ID NO: 4 or SEQ ID NO: 7, preferably by SEQ ID NO: 4. The amino acid sequence of the 22 most C-terminal amino acids of the human IL-3 amino acid sequence is provided by SEQ ID NO: 5.

At some occasions, the present application refers to a situation where binding of a molecule A to IL-3 "prevents IL-3 from activating its receptor". This relates to a situation where, upon binding of said molecule A to an IL-3 protein molecule, said IL-3 protein molecule is not capable of inducing activation of its physiological receptor anymore. Preferably, the physiological receptor of IL-3 is the IL-3 receptor. "Activation" of the IL-3 receptor denotes the molecular processes which an IL-3 receptor undergoes upon binding of IL-3 that result in transduction of a signal to the interior of an IL-3 receptor-bearing cell to bring about changes in cellular physiology. Such changes in cellular physiology in response to IL-3 receptor activation are typically activation of the JAK-STAT pathway, the Ras-MAP kinase pathway and the PI-3 kinase pathway (Oncogene 200, 19:2532-2547). IL-3 is known to activate e.g. basophils, monocytes, dendritic cells, B cells, T cells, endothelial cells. Whether a molecule prevents IL-3 from activating its receptor, can be examined by inhibition of IL-3 induced proliferation of TF-1 cells or by inhibition of IL-3 induced activation of basophils.

For several antibodies it is known that their binding to human IL-3 prevents IL-3 from activating its receptor, for example monoclonal anti-IL-3 antibodies F14-570, F14-746, F13-267 and F15-216 as published in J Immunol. 1991 Feb 1;146(3):893-8; monoclonal anti-IL-3 antibody Clone 11.14.6 and monoclonal anti-IL-3 antibody Clone 13.4.4 as described in European Patent Application No. EP12169799.9 (filing date: May 29, 2012) and international patent application PCT/EP2013/061121 (accessible through DSMZ depositions DSMACC3163 and DSMACC3164, respectively); monoclonal anti-IL-3 antibody Clone 4815 from R&D Systems (Catalog Number MAB603) and monoclonal anti-IL-3 antibody Clone 4806 from R&D Systems (Catalog Number MAB203) (see also antibody datasheets at http://www.rndsystems.com/pdf/mab603.pdf and http://www.rndsystems.com/pdf/ mab203.pdf).

Moreover, it is known that the binding to human IL-3 of antibodies binding to certain epitopes within human IL-3 prevents IL-3 from activating its receptor, for example antibodies binding to the region of
a) residues 12-15 of the human IL-3 amino acid sequence (see European Patent Application No. EP12169799.9 (filing date: May 29, 2012) and international patent application PCT/EP2013/061121),
b) residues 29-50 of the human IL-3 amino acid sequence (see J Immunol. 1991 Feb 1;146(3):893-8),
c) the 18 most N-terminal amino acids of the human IL-3 amino acid sequence (see EMBO J. 1991 Aug;10(8):2125-31), or
d) the 22 most C-terminal amino acids of the human IL-3 amino acid sequence (see EMBO J. 1991 Aug;10(8):2125-31).

At some instances, the present application refers to a certain disease being "characterized by an increased plasma IL-3 level compared to a healthy state". Such an increased plasma IL-3 level compared to a healthy state can be detected by obtaining a plasma sample from the patient considered for treatment (in the absence of any treatment with an antibody according to the invention) according to methods known in the art, determining the level of IL-3 in this plasma sample by IL-3 ELISA, and comparing the value obtained with values that are typically obtained by the same assay with plasma samples from healthy individuals (i.e. individuals that are free of any disease that may affect IL-3 levels, preferably free of any disease).

In some situations, the present application may refer to a situation where a certain compound is capable of "decreasing the plasma level of unbound IL-3" in a patient. Such capability can be determined by obtaining a plasma sample from said patient before and after administration of said compound by methods known to the person of skill in the art, determining the level of IL-3 in these plasma samples by IL-3 ELISA or carrying out functional tests, and comparing the results obtained for both samples.

At some instances, the application may refer to a patient "who has an increased plasma level of IL-3 compared to a healthy individual". Such increased plasma level of IL-3 compared to a healthy individual can be detected by obtaining a plasma sample from said patient (in the absence of any treatment with an antibody according to the invention), determining the level of IL-3 in this plasma sample by IL-3 ELISA, and comparing the value obtained with typical values as obtained by the same assay with plasma samples from healthy individuals (i.e. individuals that are free of any disease that may affect plasma levels of IL-3, preferably free of any disease).

At some instances, the present application refers to a percentage to which a first amino acid sequence is "identical" to a second amino acid sequence. This percentage is determined by aligning the two amino acid sequences using appropriate algorithms, which are known to the person skilled in the art, using default parameters; determining the number of identical amino acids in the aligned portion(s); dividing that number by the total number of amino acids in the second amino acid sequence; and then multiplying the resulting number by 100 to obtain the percentage of identical amino acids.

As used herein, the term "autoimmune disease" designates a disease resulting from an immune response against a self tissue or tissue component, including both self antibody responses and cell-mediated responses.

As used herein, the term "systemic lupus erythematosus" (abbreviated "SLE") refers to an autoimmune disease of the connective tissue characterized by production of autoantibodies to nucleic acids, complement activation, immune complex (IC) deposition in the microvasculature of various organs particularly of the kidneys. SLE a chronic inflammatory disease of unknown cause that can affect the skin, joints, bone marrow and multiple organs including kidneys, lungs and nervous system.

The term "lupus nephritis", as used herein, refers to an inflammation of the kidneys caused by systemic lupus erythematosus with renal involvement. Renal involvement usually develops in the first few years of illness and is characterized by appearance of proteinuria, heamaturia and reduction of glomerular filtration rate. Renal involvement and the specific type of glomerulonephritis are diagnosed by renal biopsy.

"Multiple sclerosis" (abbreviated "MS"), as used herein, refers to the chronic and often disabling disease of the central nervous system characterized by the progressive destruction of the myelin. There are four forms of MS: relapsing-remitting multiple sclerosis (RRMS), primary progressive multiple sclerosis (PPMS), secondary progressive multiple sclerosis (SPMS) and progressive relapsing multiple sclerosis (PRMS). "Relapsing-remitting multiple sclerosis" (RRMS) is characterized by clearly defined disease relapses (also known as exacerbations) with full recovery or with sequelae and residual deficit upon recovery periods between disease relapses characterized by a lack of disease progression. The defining elements of RRMS are episodes of acute worsening of neurologic function followed by a variable degree of recovery, with a stable course between attacks. "Primary progressive multiple scleroses" (PPMS) is characterized by disease progression with unrelenting deterioration of neurological function from the onset allowing for occasional plateauing and at times minor improvements in neurological functioning. The essential element in PPMS is a gradual and almost continuously worsening function allowing for minor fluctuations but without distinct relapses. "Secondary progressive multiple sclerosis" (SPMS) is characterized by following an initial RRMS disease course with progression, with or without occasional relapses, minor remissions, and periods of stagnation or plateaus. SPMS may be seen as a long-term outcome of RRMS in that most SPMS patients initially begin with RRMS as defined above. However, once the baseline between relapses begins to progressively deteriorate, the patient has switched from RRMS to SPMS. "Progressive relapsing multiple sclerosis" (PRMS) is characterized by progressive disease from onset, with clear acute relapses, with or without full recovery periods between relapses characterized by continuing progression.

The term "treatment", as used herein, refers to the process of providing a subject with a pharmaceutical treatment, e.g., the administration of a drug, such that at least one symptom of the disease is decreased. Treatment of a disease can be improving the disease and/or curing the disease.

The phrase that something/an event occurs "simultaneously with said administration of said anti-IL-3 antibody or IL-3 binding fragment thereof" is meant to designate that the event occurs (a) at the same time at which said antibody/fragment thereof is applied to the patient, (b) at a time that lies between individual administrations of said antibody/fragment thereof, if administration of said antibody/fragment thereof occurs in several individual administrations, or (c) after administration of said antibody/fragment thereof, while the administered antibody/fragment thereof is still present in the tissue or blood of the patient.

The term "pharmaceutically acceptable carrier, diluent and/or excipient" refers to a non-toxic, inert, solid, semi-solid, or liquid diluent material or formulation auxiliary of any type. "Pharmaceutically acceptable" in this context is meant to designate that said carrier is compatible with the other ingredients of the pharmaceutical composition and not harmful to the patient that the pharmaceutical composition is administered to. Examples of pharmaceutically acceptable carriers include, but are not limited to, water, water-propylene glycol solutions, or aqueous polyethylene glycol solutions.

The term "agent effective for treatment" of a certain disease, as used herein in the context of an agent effective for treatment of a certain disease, is meant to designate an agent that, upon administration of an effective amount of said agent to a subject suffering from that disease, results in decrease of at least one symptom of said disease.

The term "effective amount", as used herein, refers to an amount that produces a desired treatment effect in a subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. A person skilled in the art will be able to determine an effective amount through routine experimentation, namely by monitoring a subject's response to administration of a compound and adjusting the dosage accordingly. For additional guidance, see Remington: The Science and Practice of Pharmacy 20th Edition, Gennaro, Ed., Williams & Wilkins Pennsylvania, 2000.

The inventors have surprisingly found that by application of an anti-IL-3 antibody systemic lupus erythematosus and multiple sclerosis can be treated.

Interleukin-3 (IL-3), together with granulocyte-macrophage colony-stimulating factor (GM-CSF) and Interleukin-5 (IL-5) belongs to a family of hematopoietic cytokines with 4 short α-helices bundles. All of these cytokines bind to a common β-receptor subunit and a unique α-receptor subunit. IL-3 is mainly produced by activated CD4⁺ T cells (but can also be expressed by neurons) and promotes the differentiation of basophils and mast cells in the bone marrow and supports survival, growth and differentiation of CD34⁺ hematopoietic progenitor cells. Together with interferon-β (IFNβ) or IL-4, IL-3 supports differentiation of monocytes into dendritic cells. IL-3 plays an important role during parasite infection by increasing the numbers of basophils and tissue mast cells. It has been reported that IL-3 induces and facilitates histamine and IL-4 release from basophils.

IL-3 exerts its biological activities through binding to a specific cell surface receptor. The high affinity receptor responsible for IL-3 signaling is composed of alpha and beta subunits. The IL-3 receptor alpha subunit is a member of the cytokine receptor super family and binds IL-3 with low affinity. Two distinct beta subunits, AIC2A (beta_{IL-3}) and AIC2B (beta_{c}) are present in mouse cells. Beta_{IL-3} also binds IL-3 with low affinity and forms a high affinity receptor with the alpha subunit. The beta_{c} subunit does not bind any cytokine but forms functional high affinity receptors with the alpha subunit of the IL-3, IL-5 and GM-CSF receptors. Receptors for IL-3 are present on bone marrow progenitors, macrophages, mast cells, eosinophils, megakaryocytes, basophils, endothelial cells, B cells, T cells and various myeloid leukemic cells.

It has been found through mutagenesis studies that amino acids S17, N18, D21, E22, T25, E43, M49, R94, P96, R108, F113, and E119 of human IL-3 are critical residues that are solvent-exposed and important for binding of human IL-3 to the IL-3 receptor (Immunol Rev. 2012 Nov;250(1):277-302).

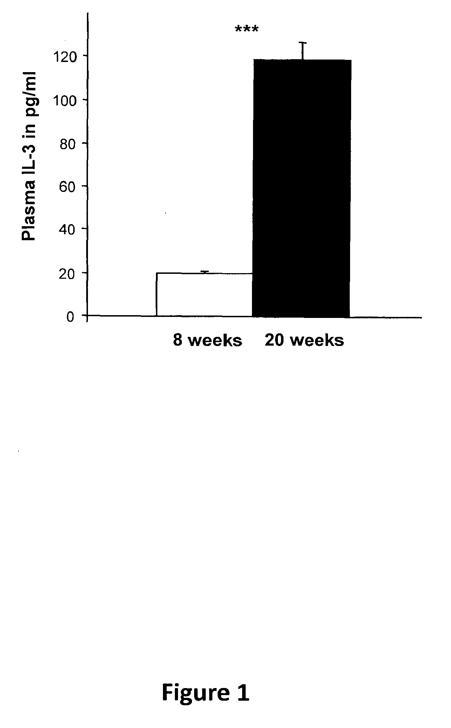

Systemic lupus erythomatosus (SLE) is an autoimmune disease characterized by production of autoantibodies to nucleic acids, complement activation, immune complex (IC) deposition in the microvasculature of various organs particularly of the kidneys. The murine model of MRL/*lpr* mice develops spontaneous autoimmune disease that closely resembles human systemic lupus erythematosus and its various immunopathologic characteristics, including the appearance of autoantibodies (such as anti-dsDNA, anti-Sm and anti-myeloperoxidase antibodies), hypergammaglobulinemia, circulating immune complexes immune complex glomerulonephritis and systemic vasculitis, sialoadenitis, cytokine abnormalities like increased production of IL-1, IL-6, and inflammatory diseases of the lungs and skin. Moreover, in MRL/*lpr* mice plasma IL-3 titers increase with disease progression (see below, Fig. 1), which mirrors the situation in humans, where SLE patients feature higher serum titers of IL-3 in comparison to healthy controls (P. Fishman et al. 1993. Interleukin-3 immunoassay in systemic lupus erythematosus patients: preliminary data. Int Arch Allergy Immunol 100:215-218). The mice carry the *lpr* mutation in the apoptosis related Fas gene, which leads to accumulation of autoreactive T and B cells as well as activated macrophages.

The inventors have found that blockage of IL-3 in MRL/*lpr* mice by administration of an anti-IL-3 antibody significantly reduces renal IgG deposition, indices for activity and chronicity in kidneys and deposition of collagen I, thus reducing SLE symptoms. In contrast, administration of IL-3 leads to significantly increased renal IgG deposition, more severe lupus activity in the kidneys and increased renal fibrosis.

In addition, the inventors have made observations showing that neutralization of IL-3 also has a beneficial effect in experimental autoimmune encephalomyelitis (EAE), while injection of recombinant IL-3 results in exacerbation of EAE. These experiments were carried out with C57BL/6 mice with MOG-peptide induced EAE, an animal model for human multiple sclerosis. It has been reported that in this model system IL-3 is produced after MOG-specific restimulation of splenic CD4⁺ T cells, or total leukocytes from lymph nodes, CNS, blood and spleen (HH Hofstetter et al., The cytokine signature of MOG-specific CD4 cells in the EAE of C57BL/6 mice. J Neuroimmunol 2005; 170:105-14). This reflects the situation in humans, where transcriptional analysis of cytokine expression in brain specimens from MS-patients and healthy controls is known to result in upregulation of IL-3 expression in MS-lesions (SE Baranzini, et al. Transcriptional analysis of multiple sclerosis brain lesions reveals a complex pattern of cytokine expression. J Immunol 2000; 165:6576-82). Thus, the observations in the mouse model allow the conclusion that blocking of IL-3 is a target for treatment of human multiple sclerosis.

Based on the results obtained from the murine model, it can be expected that blockade of IL-3 in human patients with SLE will have similar effects as blockade of IL-3 in the murine lupus models in MRL/*lpr* mice. In humans with lupus nephritis it is anticipated that less cellular infiltration in the kidney, less acute and chronic damage in the kidney, reduced Ig deposition and less fibrosis will be observed.

In patients with MS, it is anticipated that blockade of IL-3 will reduce the leukocyte infiltration in the brain, especially during acute attacks, will diminish demyelination and axonal damage and therefore reduce clinical signs and complications of MS.

IL-3 deficient mice have no overt phenotype suggesting that blockade of IL-3 has much less side effects than the treatment options known from the prior art. IL-3 can be measured in the serum, plasma or cerebrospinal fluid of patients with SLE and MS and used to select certain subgroups of patients for treatment with blockade of IL-3.

In the following, reference is made to the figures:
All methods mentioned in the figure descriptions below were carried out as described in detail in the examples.
Figure 1 shows data from an *in vivo* cytokine capture experiment addressing the question whether IL-3 is produced in MLR/*lpr* mice and whether there is an increase or decrease in IL-3 production during progression of the disease. 10 µg of biotin-labelled anti-IL-3 was injected into the tail vein of 8 and 20 weeks old MRL/*lpr*. Blood was drawn 3 hours later and tested for IL-3 by ELISA. Plasma IL-3 titer increased highly significant with increasing age and disease progression (p= 8x10⁻⁵).
   *** indicates significance (p<0.001)

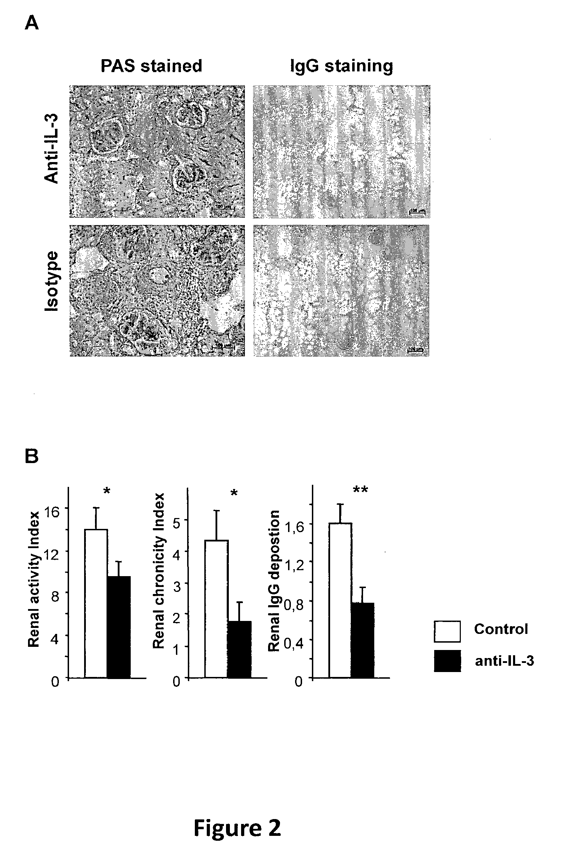

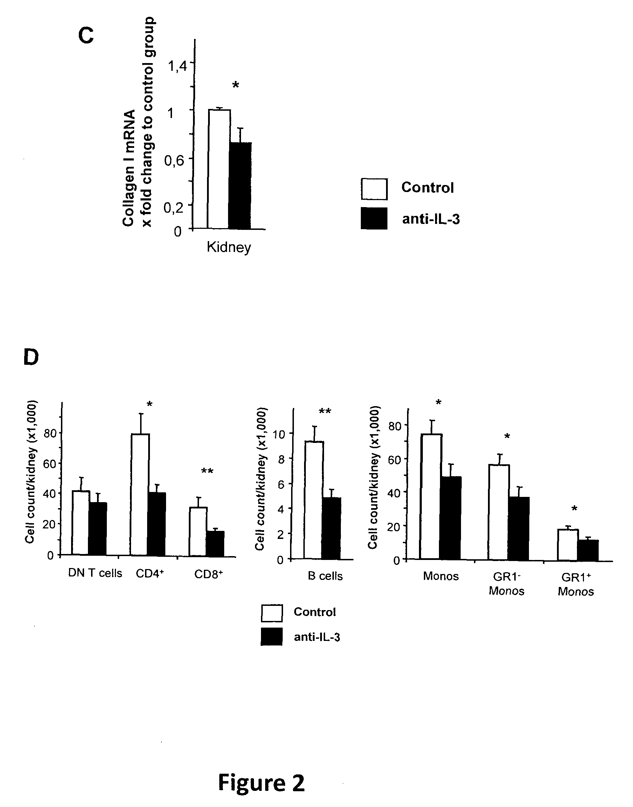

Figure 2 shows the effects of anti-IL-3 treatment in MRL/*lpr* mice as determined by histological analysis, gene expression analysis by real-time PCR, and flow cytrometric analysis.
   18 weeks old male mice (n=9) were treated with daily intraperitoneal injections of anti-IL-3 or isotype (purified rat IgG) for 4 weeks.
   A: Sections from a mouse treated with anti-IL-3 or control show less glomerular hyper cellularity, focal segmental sclerosis, global sclerosis, leukocyte infiltration, sub endothelial IgG deposition in the anti-IL-3 treated mice (IgG stained, magnification x10, periodic acid-Schiff stained, magnification x20).
   B: Summary of histological scores. Scores of IgG deposition, activity and chronicity index. Values are the mean and SEM.
   C: Expression of collagen I mRNA in the kidneys was measured by real time PCR. Collagen I was about 40 per cent less in the anti-IL-3 treated mice in comparison with the control mice.
   D: Flow cytometric analysis of the infiltrating cells in the kidney of anti-IL-3 treated mice vs. controls. There are significantly less infiltrating monocytes, monocyte subpopulations, T cells (especially CD4⁺) and B-lymphocytes in the anti-IL-3 treated group in comparison to the isotype control.
   ** indicates significance (p<0.01). * indicates significance (p<0.05).

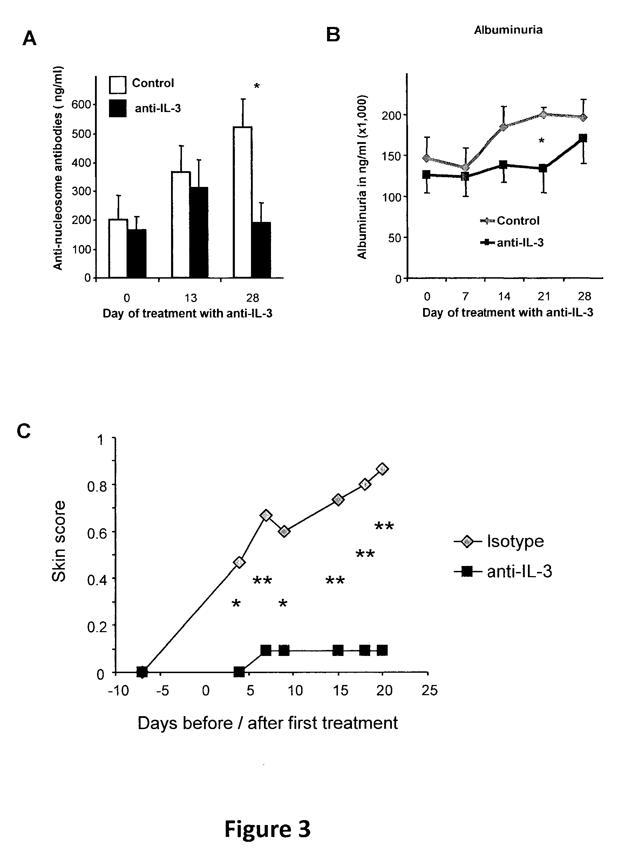

Figure 3 shows the effects of anti-IL-3 treatment in MRL/*lpr* mice on autoantibody production and albuminuria as determined by ELISA and on the formation of skin lesions.
   A, B: Mice (n=9) were treated as described in Figure 2.
   A: Anti-nucleosome autoantibodies were measured in the plasma of anti-IL-3 or isotype treated mice on day 0, 13 and 28 of treatment. No regular increase of anti-nucleosome antibody titers was observed in anit-IL-3 treated mice.
   B: Weekly spot urine was collected and albuminuria was measured by ELISA. In the anti-IL-3 group albuminuria remained stable until day 21 of treatment, while there was a significant increase in the control group.
   C: 16 weeks old male mice received daily (7 days/week) i.p. injections of 100 µg anti-IL-3 antibody (n=11) or purified rat IgG (n=13) for 3 weeks. Treatment with anti-IL-3 significantly reduced the development of lupus like skin lesions.
   ** indicates significance (p<0.01). * indicates significance (p<0.05).

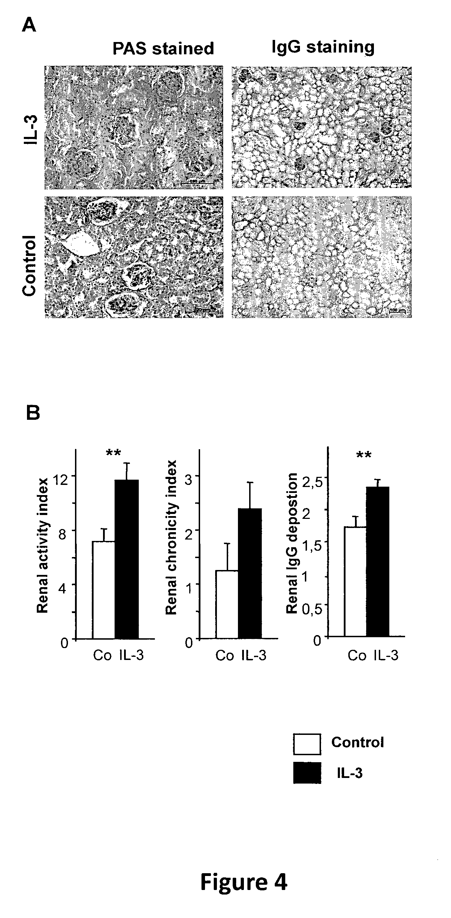

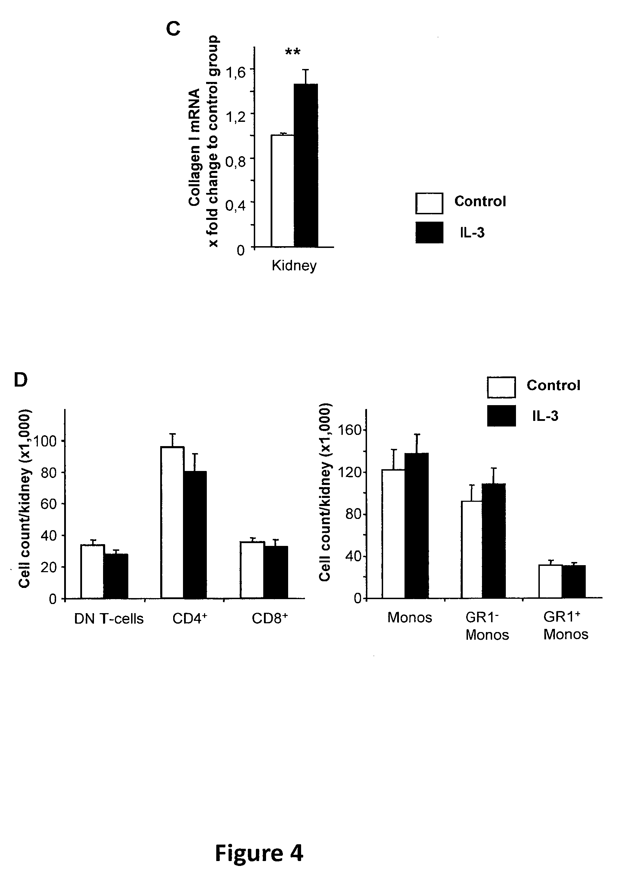

Figure 4 shows the effects of IL-3 administration on development of lupus nephritis in MRL/*lpr* as determined by histological, real-time PCR and flow cytrometric analysis. 16 weeks old male mice (n=13) were treated with daily intraperitoneal injections of IL-3 or control (PBS) for 3 weeks.
   A: Sections from a mouse treated with IL-3 or control. More glomerular hyper cellularity, focal segmental sclerosis, global sclerosis, leukocyte infiltration, sub endothelial IgG deposition are seen in IL-3 treated mice than in the PBS treated control (IgG stained, magnification x10, periodic acid-Schiff stained, magnification x20).
   B: Summary of histological scores. Scores of IgG deposition, activity and chronicity index. Values are the mean and SEM.
   C: Collagen I in the kidneys was measured by real time PCR and was increased by about 45 per cent in the IL-3 treated mice.
   D: Flow cytometric analysis of cells infiltrating the kidney of IL-3 treated mice and controls.
   ** indicates significance (p<0.01).

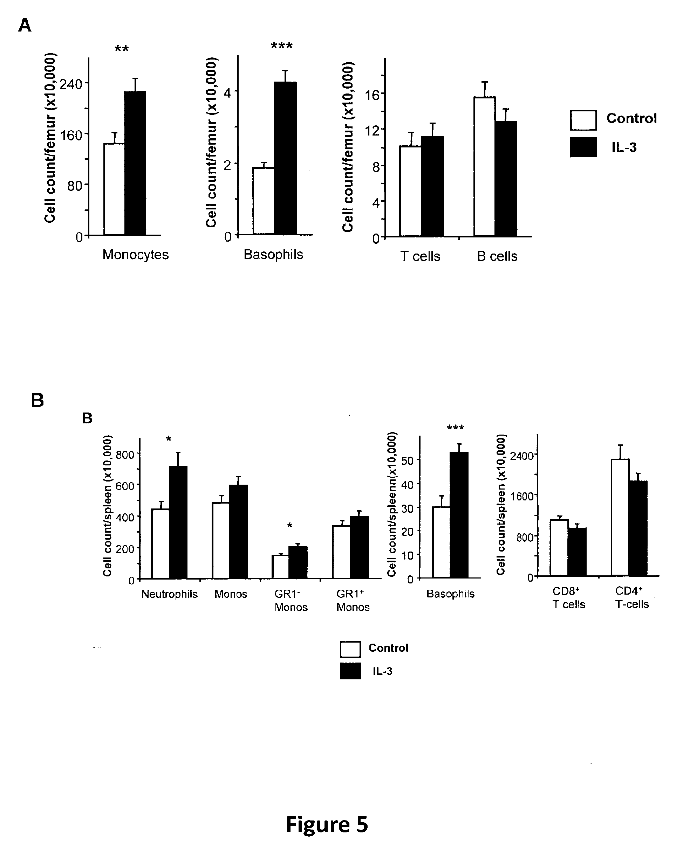

Figure 5 shows the effects of IL-3 on development of SLE in MRL/*lpr* mice as determined by flow cytometry.
   Mice (n=13) were treated as described in Figure 4.
   Monocytes, basophils, B-and T cells were identified by flow cytometric analysis in the spleen (A) and bone marrow (B) of mice treated with IL-3 or PBS. No significant differences in the number of T cells including CD4⁺ and CD8⁺ T cells in spleen and bone marrow were found. The numbers of monocytes and basophils were significantly increased in the spleen and bone marrow. Furthermore neutrophils and the GR-1⁺ monocyte subpopulation were significantly increased in IL-3 treated mice.
   *** indicates significance (p<0.001). ** indicates significance (p<0.01). * indicates significance (p<0.05).
Figure 6 shows the gating strategy of the flow cytometric analysis of the kidneys.
Figure 7 shows the gating strategy of the flow cytometric analysis of the spleen.
Figure 8 shows the gating strategy of the flow cytometric analysis of the bone marrow.
Figure 9 shows the effects of IL-3 application and of application of the anti-IL-3 antibody MP2-8F8 on IL-4 release by DX5-positive cells as determined by ELISA.
   IL-3 induces a pronounced release of IL-4 from DX5⁺ cells. Pre-incubation of IL-3 with the anti-IL-3 antibody MP2-8F8 prevents the IL-3 induced release of IL-4. Thus, the anti-IL-3 antibody MP2-8F8 has a neutralizing activity on IL-3.
Figure 10 displays data from clinical severity scoring and flow cytometric analysis showing that blockade of IL-3 with a monoclonal antibody reduces development of EAE (daily i.p. treatment with anti-IL-3 (50 µg) from day 0-19).
   EAE was induced in C57BL/6 mice by immunization with MOG-peptide on day 0. Mice were treated by daily i.p. injection of 50 µg of a neutralizing anti-IL-3 antibody (anti-IL-3), 50 µg of the deglycosylated anti-IL-3 antibody (Deglycosylated anti-IL-3) or the same amount of purified rat IgG (Control) from day 0-19.
   A, D: Clinical symptoms of EAE (EAE score) are highly significantly lower (p<0.01) in anti-IL-3 treated mice.
   B: The number of leukocyte subpopulations infiltrating the brain was quantified by flow cytometry on day 20. CD45⁺ indicates the total number of CD45⁺ leukocytes, CD19⁺. CD8⁺, CD4⁺ and CD11b⁺ indicates the number of CD19⁺ B cells, CD8⁺ T cells, CD4⁺ T cells and CD11b⁺ monocytes respectively.
   C: The number of leukocyte subpopulations in the peripheral blood was quantified by flow cytometry on day 20. CD19⁺. CD8⁺ and CD4⁺ indicates the number of CD19⁺ B cells, CD8⁺ T cells and CD4⁺ T cells respectively. Monos and PMN indicates the number of monocytes and neutrophils.
   ** indicates significance (p<0.01). * indicates significance (p<0.05).

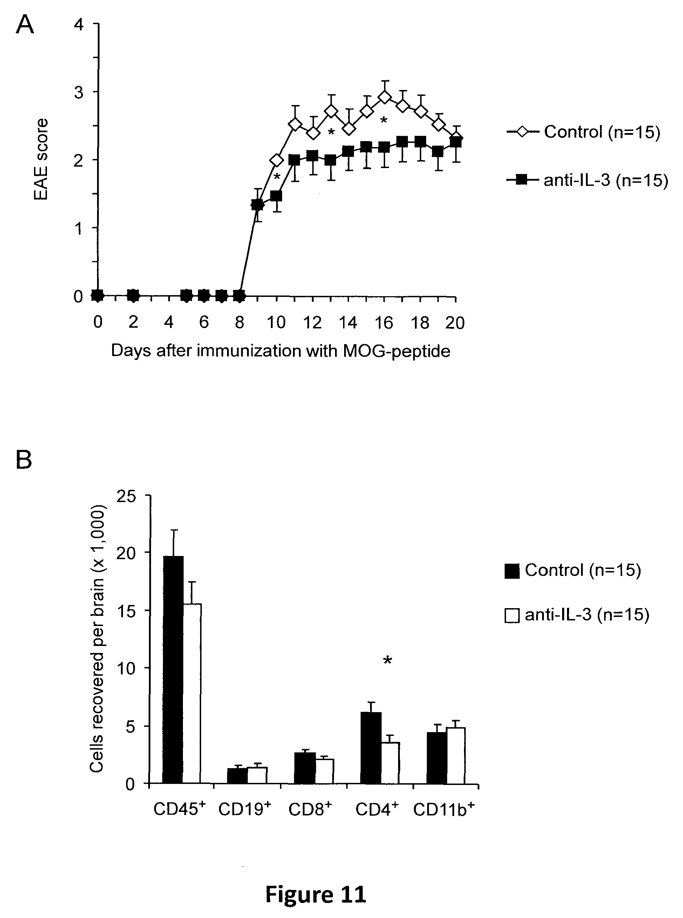

Figure 11 depicts data from clinical severity scoring and flow cytometric analysis showing that blockade of IL-3 with a monoclonal antibody reduces development of EAE (daily i.p. treatment with anti-IL-3 (50 µg) from day 5-19).
   EAE was induced in C57BL/6 mice by immunization with MOG-peptide on day 0. Mice were treated by daily i.p. injection of 50 µg of a neutralizing anti-IL-3 antibody (anti-IL-3) or the same amount of purified rat IgG (Control) from day 5-19.
   A: Clinical symptoms of EAE (EAE score) are lower in anti-IL-3 treated mice.
   B: The number of leukocyte subpopulations infiltrating the brain was quantified by flow cytometry on day 20.
   * indicates significance (p<0.05).

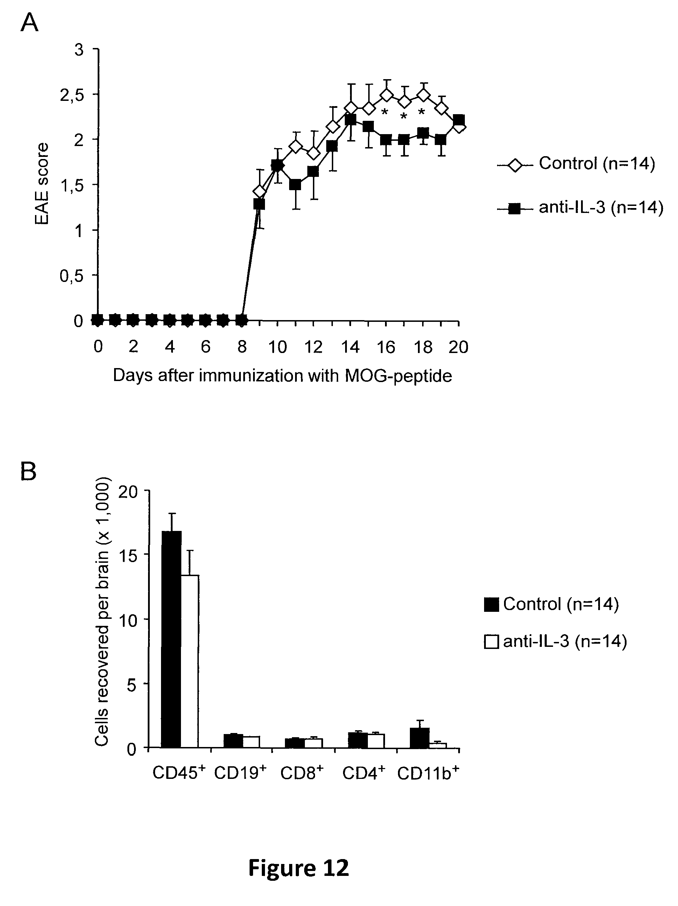

Figure 12 depicts data from clinical severity scoring and flow cytometric analysis showing that blockade of IL-3 with a monoclonal antibody reduces development of EAE (daily i.p. treatment with anti-IL-3 (50 µg) from day 10-19).
   EAE was induced in C57BL/6 mice by immunization with MOG-peptide on day 0. Mice were treated by daily i.p. injection of 50 µg of a neutralizing anti-IL-3 antibody (anti-IL-3) or the same amount of purified rat IgG (Control) from day 10-19.
   A: Clinical symptoms of EAE (EAE score) are lower in anti-IL-3 treated mice.
   B: The number of leukocyte subpopulations infiltrating the brain was quantified by flow cytometry on day 20.
   * indicates significance (p<0.05).

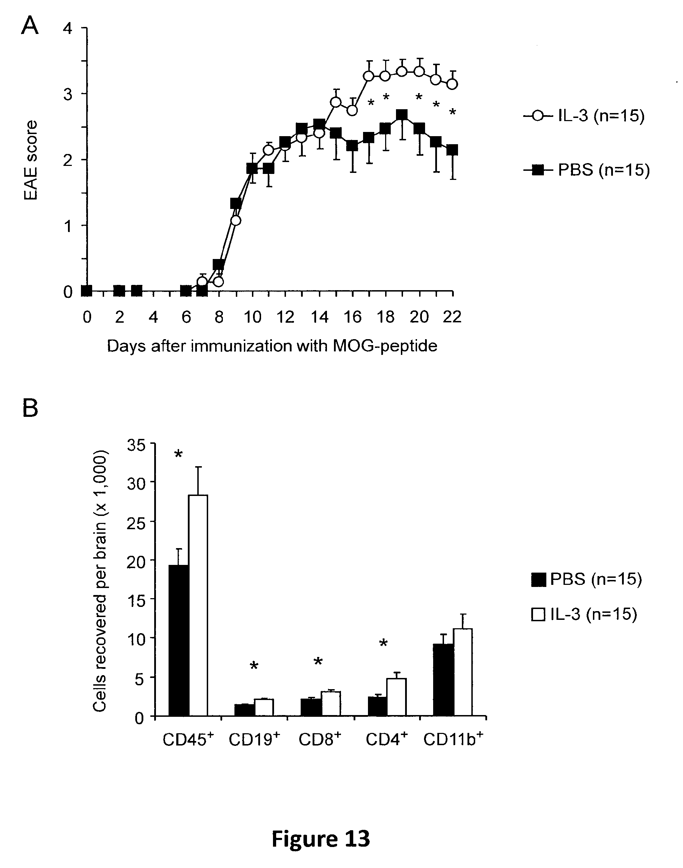

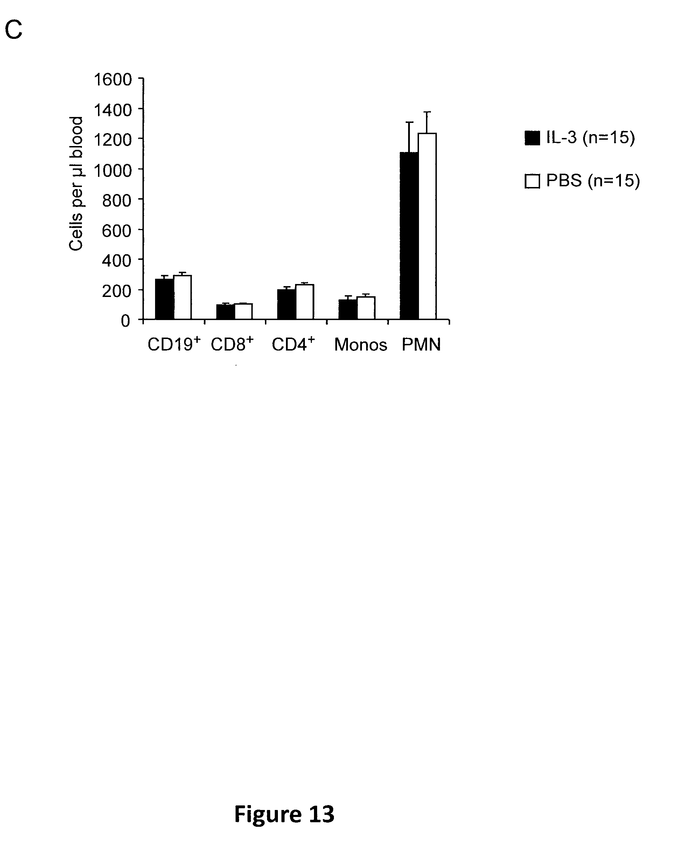

Figure 13 displays data from clinical severity scoring and flow cytometric analysis showing that injection of recombinant IL-3 exacerbates development of EAE.
   EAE was induced in C57BL/6 mice by immunization with MOG-peptide on day 0. Mice were treated by daily i.p. injection of 200 ng recombinant IL-3 (IL-3) or PBS as control (PBS) from day 5-21.
   A: Clinical symptoms of EAE (EAE score) are significantly higher in IL-3 treated mice.
   B: The number of leukocyte subpopulations infiltrating the brain was quantified by flow cytometry on day 22.
   C: The number of leukocyte subpopulations in the peripheral blood was quantified by flow cytometry on day 22.
   * indicates significance (p<0.05).
Figure 14 shows the amino acid sequence of human IL-3 and various fragments thereof.
   A: SEQ ID NO: 1: Amino acid sequence of human IL-3.
   B: SEQ ID NO: 2: Amino acid sequence of residues 12-15 of the human IL-3 amino acid sequence according to SEQ ID NO: 1.
   C: SEQ ID NO: 3: Amino acid sequence of residues 29-50 of the human IL-3 amino acid sequence.
   D: SEQ ID NO: 4: Amino acid sequence of the 18 most N-terminal amino acids of the human IL-3 amino acid sequence according to SEQ ID NO: 1.
   E: SEQ ID NO: 5: Amino acid sequence of the 22 most C-terminal amino acids of the human IL-3 amino acid sequence.
   F: SEQ ID NO: 6: Amino acid sequence of human IL-3 (alternative allele).
   G: SEQ ID NO: 7: Amino acid sequence of the 18 most N-terminal amino acids of the human IL-3 amino acid sequence according to SEQ ID NO: 6.
   H: Amino acid sequence of residues 17-133 of the human IL-3 amino acid sequence.
   I: Amino acid sequence of residues 21-133 of the human IL-3 amino acid sequence.

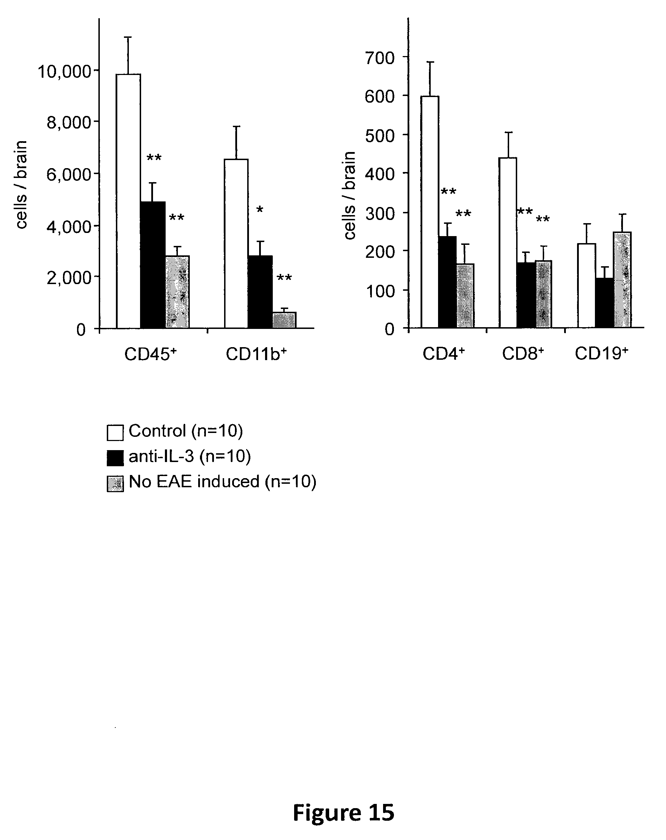

Figure 15 shows the reduction of the leukocyte infiltration of the brain after blockade of IL-3 with a monoclonal antibody.

In the following, reference is made to the examples, which are given to illustrate, not to limit the present invention.

### Examples

### Example 1

### Animals

6-8 weeks old male MRL/*lpr* mice were obtained from Harlan Winkelmann GmbH and maintained under SPF (specific pathogen free) conditions.

### Treatment of mice

18 weeks old male mice received daily (6 days/week) i.p. injections of 50 µg of a blocking IL-3 antibody (clone MP2-8F8) or purified rat IgG (Sigma-Aldrich) for 4 weeks. Alternatively 16 weeks old male mice received daily (7 days/week) i.p. injections of 100 µg anti-IL-3 antibody (clone MP2-8F8) or purified rat IgG (Sigma-Aldrich) for 3 weeks.

To investigate the effects of IL-3 on the development of lupus nephritis (an inflammation of the kidney caused by systemic lupus erythematosus), 16 weeks old male mice were treated daily (6 days/week) by i.p. injections of 200 ng recombinant IL-3 (PeproTech, Rocky Hill, NJ) or phosphate buffered saline (PBS) for 3 weeks.

### Lupus skin score

The lupus skin score was evaluated before and at several time points after treatment of 16 weeks old mice with anti-IL-3 or isotype control antibody. The skin lesions were scored by gross pathology using a grade of 0 to 2 (0 = none; 1 = lesions on snout, ears and tail; 2 = lesions on the back).

### ELISA and in vivo cytokine capture

10 µg of biotin-conjugated anti-IL-3 (MP2-43D11, rat, IgG2a, k, Biolegend) was injected in 200 µl sterile PBS in the tail vein of 8 and 20 weeks old MRL/*lpr* mice. After 3 hours blood was drawn from anesthetized mice by retro bulbar puncture. Samples were stored until use at -20°C. Plasma IL-3 was then measured by ELISA, according to manufacturer's protocol (BD OptEIA).

Concentration of albumin in the urine was measured by ELISA (Bethyl Laboratories).

### Evaluation of glomerulonephritis

Kidneys were fixed in 10% buffered formalin and embedded in paraffin. Five-micrometer sections for periodic acid-Schiff stain (PAS) were prepared following routine protocols. The severity of the renal lesions was graded from 0 to 3 using the indices for activity and chronicity as described for human lupus nephritis (H. A. Austin et al. 1984. Diffuse proliferative lupus nephritis: identification of specific pathologic features affecting renal outcome. Kidney Int 25:689-695). The severity of the glomerular IgG deposits was graded semi quantitative from 0 to 4. All grading was done by a blinded observer.

### Flow cytometry and antibodies

The following fluorochrome labeled antibodies were used for flow cytometry and obtained from BD Biosciences or eBioscience: anti-CD11b (M1/70), anti-FceRI (MAR-1), anti-Ly6G/Ly6C (RB6-8c5), anti-F4/80 (BM8), anti-CD19 (eBio1D3), anti-CD8 (53-6.7), anti-CD3e (145-2C11), anti-CD3 (eBio500A2) anti-CD4 (RM4-5), anti-CD45 (30-F11), anti-CD49b (DX5). Unfixed cells from kidney, spleen or bone marrow were pre-incubated for 10 minutes with anti-CD16/32 (2.G4.2, rat, IgG2b,k, BD) at room temperature and then for 20 minutes at 4°C with combinations of labeled antibodies. After one washing step, red blood cells were lysed with FACS lysing solution (BD Biosciences) and samples were analyzed on a FACSCanto II with FACSDIVA software (BD Biosciences).

### Lupus autoantibodies

Serum antibody levels were determined by Anti-nucleosome ELISA: NUNC maxisorp ELISA plates were coated with histones (5 µg/ml) and mouse embryonic stem cell dsDNA (1 µg/ml) overnight. Prior to the coating of the sample wells with histones and dsDNA, plates were layered with poly-L-lysin (Trevigen) for 1 h at room temperature followed by washing with wash buffer. After overnight coating with histones and dsDNA, serum samples were analyzed for anti-nucleosome IgG by using mouse IgG detection kit (Bethyl Labs). Reference serum with specific IgG was used as a positive control and to calculate autoantibody concentrations.

### Reverse transcription and real-time PCR

mRNA and total protein were isolated from kidneys by the RNeasy Midi Kit (Qiagen). Total RNA was reversely-transcribed with oligo(dT) and M-MLV reverse transcriptase (Invitrogen). Real-time PCR was performed on a TaqMan Vii A7 using QuantiTect SYBR Green PCR kit (Qiagen). Data were analyzed with ViiA7v1.2.1 software (Applied Biosystems). Sequences of primers were: Collagen I: 5'-TGT TCA GCT TTG TGG ACC TC-3' (forward) and 5'-TCA AGC ATA CCT CGG GTT TC-3' (reverse). Controls consisting of ddH₂0 (double-distilled water) were negative for target and housekeeper gene, HPRT.

### Statistical analysis

Mean ± SEM values were calculated. Significance of group differences was determined by Student's 1-sided t-Test. P values less than 0.05 were considered significant and marked with one asterisk, if less than 0.05, or with two asterisks, if less than 0.01. P values less than 0.001 were marked with three asterisks.

### Example 2

### IL-3 levels in MLR/lpr mice

All methods mentioned in this example were carried out as described in Example 1.

In this Example, it was examined whether IL-3 is produced in MLR/*lpr* mice and whether there is an increase or decrease in IL-3 production during progression of the disease. IL-3 levels in plasma were measured by *in vivo* cytokine capture in 8 and 20 weeks old MRL/*lpr* mice (n=4/ time point). At 8 weeks, when mice do not show any signs of systemic lupus plasma IL-3 levels were low (20 pg/ml) but significantly increased until week 20 (118 pg/ml) (Figure 1).

### Example 3

Treatment with monoclonal IL-3 antibody in early systemic lupus ameliorates lupus nephritis

All methods mentioned in this example were carried out as described in Example 1.

To investigate, whether increased IL-3 levels contribute to development of lupus nephritis, mice were treated with a blocking antibody against IL-3. 18 weeks old male mice (n=9) received daily i.p. injections of 50 µg anti-IL-3 antibody (clone MP2-8F8), while the control group received the same amount of a control antibody (n=9). Mice were treated for 28 days and analyzed one day after the last injection. Histological analysis of the kidneys showed a significantly lower score for lupus activity and chronic damage in anti-IL-3 treated animals. In addition, significantly less IgG depositions were found in the kidneys of the anti-IL-3 treated group (Figure 2A,B).

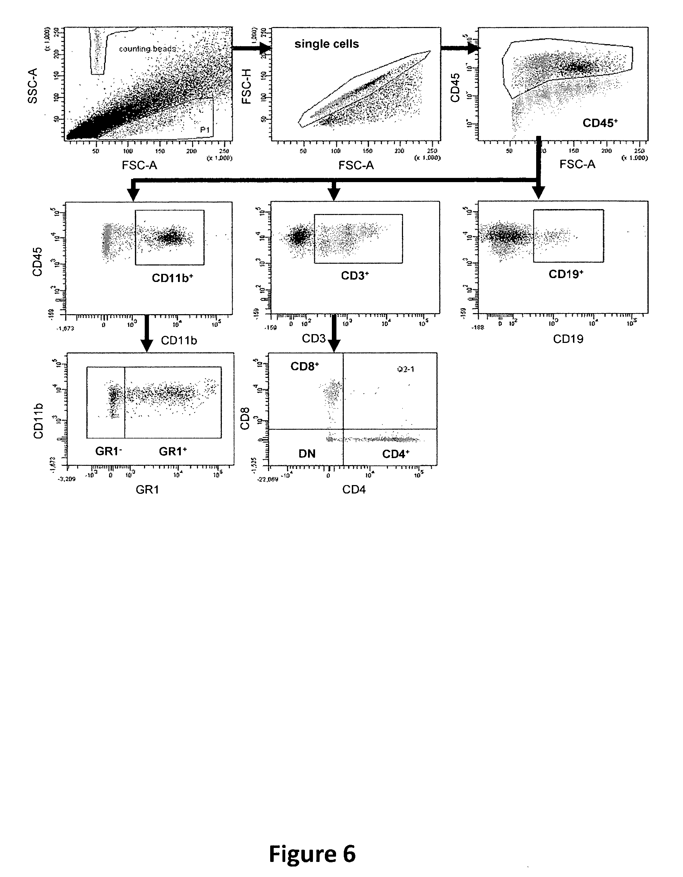

Flow cytometric analysis of single cell suspension of the kidneys (gating strategy shown in Fig. 6) revealed a significant decrease of infiltrating CD11b⁺ monocytes in anti-IL-3 treated mice. Both Gr1⁺ and Gr1⁻ monocytes were reduced. Moreover, infiltration of the kidneys with CD4⁺, CD8⁺ T cells as well as CD19⁺ B cells was significantly reduced. (Figure 2 D).

Renal fibrosis measured by real-time PCR of collagen I was decreased by 30% in the anti-IL-3 treated mice (Figure 2 C).

### Example 4

### Anti-IL-3 treatment decreases urinary albuminuria and lupus autoantibodies in the plasma

All methods mentioned in this example were carried out as described in Example 1.

Weekly spot urine was collected and albuminuria was measured by ELISA. In the anti-IL-3 group albuminuria remained roughly stable until day 21 of treatment and was significantly lower than in the control group. At the end of the experiment (day 28) differences were no longer significant (Figure 3 B).

Blood was drawn weekly to measure autoantibodies by ELISAs. There were no significant differences in the amount of Smith or dsDNA autoantibodies. However anti-nucleosome autoantibodies were significantly less in the anti-IL-3 treated mice at day 28 of treatment. In the control group was a continuous increase of anti-nucleosome antibody from day 0 to 28 (Figure 3 A).

### Example 5

### Anti-IL-3 treatment decreases lupus manifestations on the skin

All methods mentioned in this example were carried out as described in Example 1.

16 weeks old male mice received daily (7 days/week) i.p. injections of 100 µg anti-IL-3 antibody (clone MP2-8F8) or purified rat IgG (Sigma-Aldrich) for 3 weeks. The skin score was evaluated on a scale of 0-2. Mice treated with control antibodies developed pronounced lupus-like skin lesions while treatment with anti-IL-3 almost completely prevented development of skin lesions (Fig. 3C).

### Example 6

### Administration of IL-3 aggravates lupus nephritis

All methods mentioned in this example were carried out as described in Example 1.

In this Example, it was investigated whether administration of IL-3 during progression of disease onset increases the incidence and severity of glomerulonephritis. 16 weeks old male mice (n=12) were treated for 21 days with daily i.p. injections of 100 ng recombinant IL-3, while the control group (n=13) received the same volume of PBS. Application of IL-3 significantly increased the histological score for activity and IgG depositions in the kidneys (Figure 4A,B). There was also a trend to a higher chronicity index in the kidneys of IL-3 treated mice. Flow cytometric analysis revealed no significant differences in the numbers of cells infiltrating the kidneys (CD4⁺, CD8⁺ or double negative T cells; gating strategy shown in Fig. 6) (Figure 4 D). Renal fibrosis measured by real-time PCR of collagen I was increased by 45 % in IL-3 treated mice (Figure 4 C).

### Example 7

### IL-3 administration increases urinary albuminuria and lupus autoantibodies in the plasma

All methods mentioned in this example were carried out as described in Example 1.

Weekly spot urine was collected and albuminuria was measured by ELISA. There was no significant difference in the albuminuria between the IL-3 treated and the control group.

Under administration of IL-3 no significant difference between the titers of Smith, dsDNA or nucleosome autoantibodies was detected.

### Example 8

### Administration of IL-3 causes an increase of monocytes and basophils in spleen and bone marrow

All methods mentioned in this example were carried out as described in Example 1.

16 weeks old male mice (n=13) were treated with daily intraperitoneal injections of IL-3 or control (PBS) for 3 weeks.

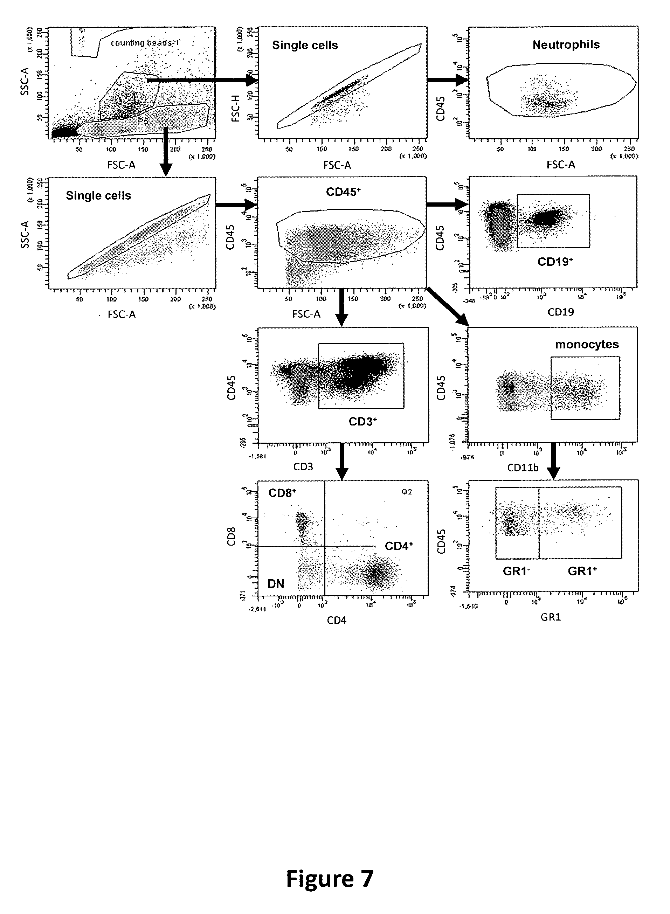

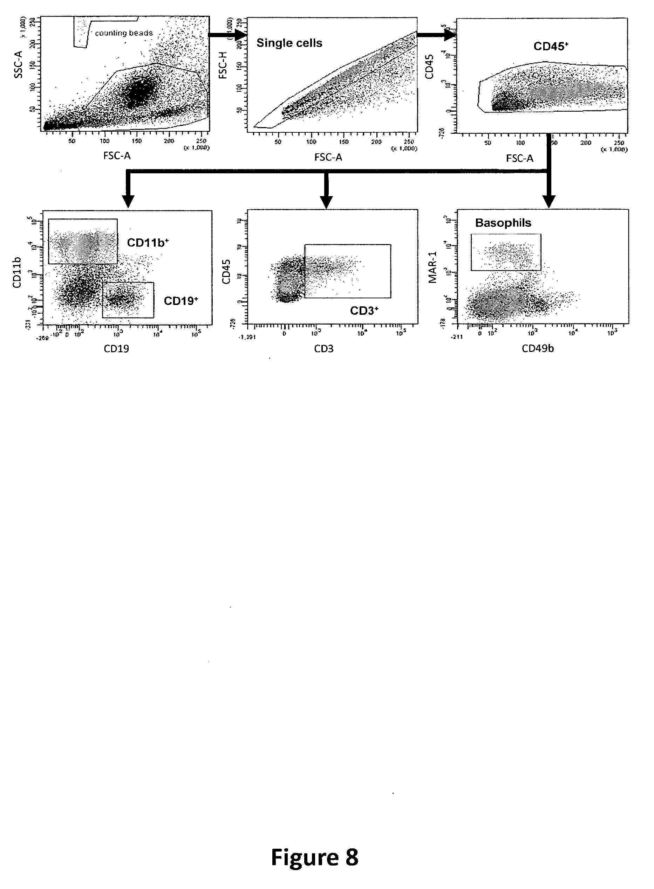

In flow cytometric analyses, it was found that injection of IL-3 increases the numbers of basophils, CD11b⁺ monocytes and neutrophils in spleen and bone marrow (Figure 5; gating strategies shown in Figures 7-8). No difference in T cells, T cell subpopulations or B cells in the bone marrow or spleen was observed.

### Example 9

### Neutralizing activity of the anti-IL-3 antibody MP2-8F8

DX5 positive cells (mainly basophils) were isolated from the bone marrow of C57BL/6 mice using magnetic beads (Miltenyi). Recombinant mouse IL-3 (1 ng/ml) was pre-incubated with various concentrations of anti-IL-3 antibody (MP2-8F8) for 30 min at room temperature. Then, DX5⁺ cells were added (20.000 cells / well) and incubated in a total volume of 200 µl for 24 h. IL-4 was measured in the supernatant with a commercial ELISA from Becton-Dickinson.

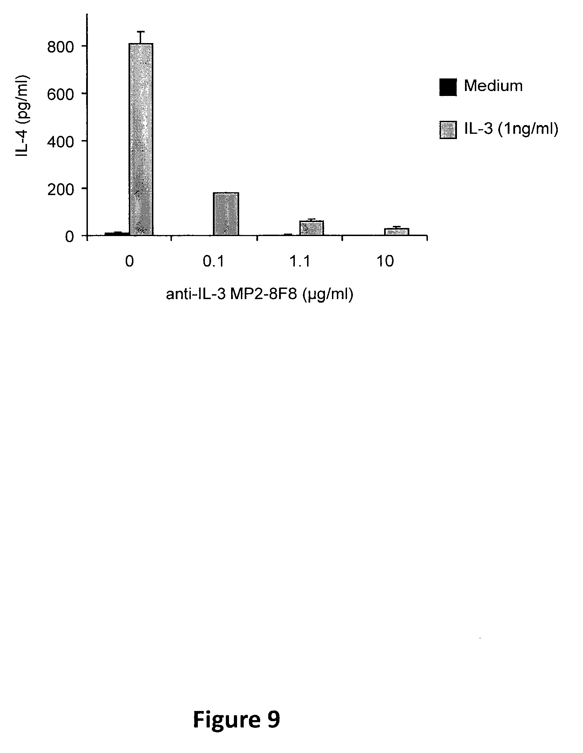

As can be seen from the data depicted in Figure 9, IL-3 induces a pronounced release of IL-4 from DX5⁺ cells. Pre-incubation of IL-3 with the indicated concentrations of anti-IL-3 antibody MP2-8F8 prevents the IL-3 induced release of IL-4.

### Example 10

### Induction of Experimental autoimmune encephalomyelitis (EAE)

On day 0 female 8-12 weeks old C57BL/6 mice were immunized subcutaneously at both flanks with a total of 100 µl solution containing 200 µg MOG-peptide (MEVGWYRSPFSRVVHLYRNGK) in complete Freund's adjuvant (Sigma F5506) containing 1 mg *M butyricum* (Becton Dickinson 264010). On days 0 and 2 mice were injected i.p. with 0.25 µg pertussis toxin from *B. pertussis* (Sigma P7208) dissolved in 200 µl PBS containing 1% bovine serum albumin. Individual animals were observed daily and clinical scores were assessed by a blinded investigator as follows: 0 = no clinical disease, 1 = loss of tail tone only, 2 = partial paralysis of hind legs, 3 = complete paralysis of hind legs and one front leg, 4 = complete paralysis of hind legs and partial or complete paralysis of both front legs, and 5 = complete paralysis of all legs or death.

### Treatment of mice

Mice were treated as indicated in the figure legends by daily i.p. injection of 50 µg purified anti-IL-3 antibody (clone MP2-8F8, Biozol), 50 µg purified and deglycosylated anti-IL-3 antibody or the same amount of purified rat IgG (Jackson Immunoresearch). Alternatively, mice were treated by daily i.p. injection of 200 ng recombinant IL-3 or PBS as control. To deglycosylate the anti-IL-3 antibody, a digestion with PNGase (New England Biolabs) was performed followed by dialysis of the antibody against PBS.

### Flow cytometry of peripheral blood and brain tissue

Peripheral blood was drawn from the retroorbital venous plexus of anesthetized mice and anticoagulated with EDTA. Single cell suspensions of brain tissue were prepared as follows. Half of the brain was cut into small pieces and pressed through a 100 µm cell strainer in a total volume of 1 ml. After centrifugation cells were resuspended in 8 ml 40% Percoll. 2 ml 80% Percoll was underlayed and centrifuged for 20 min at 2,000 rpm. Cells in the interphase were recovered and washed once in RPMI-medium with 10% FCS. For flow cytometry, cells were pre-incubated for 10 min on ice with Fc-block (clone 2.4G2; 5 µg/ml) and then stained with combinations of directly labelled antibodies for 25 min. The following antibodies were obtained from BD Bioscience and eBioscience: anti-CD4 (clone RM4-5), anti-CD8 (clone 53-6.7), anti-CD 19 (clone eBio1D3), anti-CD11b (clone M1/70), anti-CD45 (cone 30-F11), anti-Ly-6G (clone 1A8). Red blood cells were lysed with FACS-lysing solution (BD Biosciences) and samples analyzed on a FACSCantoII (BD Biosciences) with FlowJo software (Tree Star). For analysis, leukocytes were first gated according to their FSC-SSC properties and expression of surface markers shown on total leukocytes. The number of cells was quantified using counting beads (Invitrogen).

### Example 11

All methods mentioned in this example were carried out as described in Example 10.

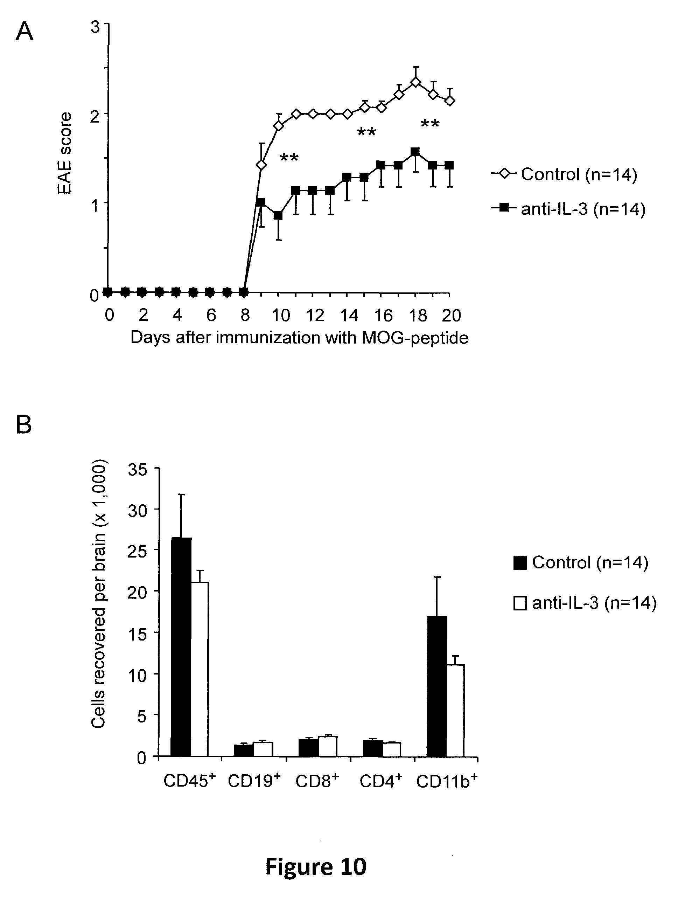

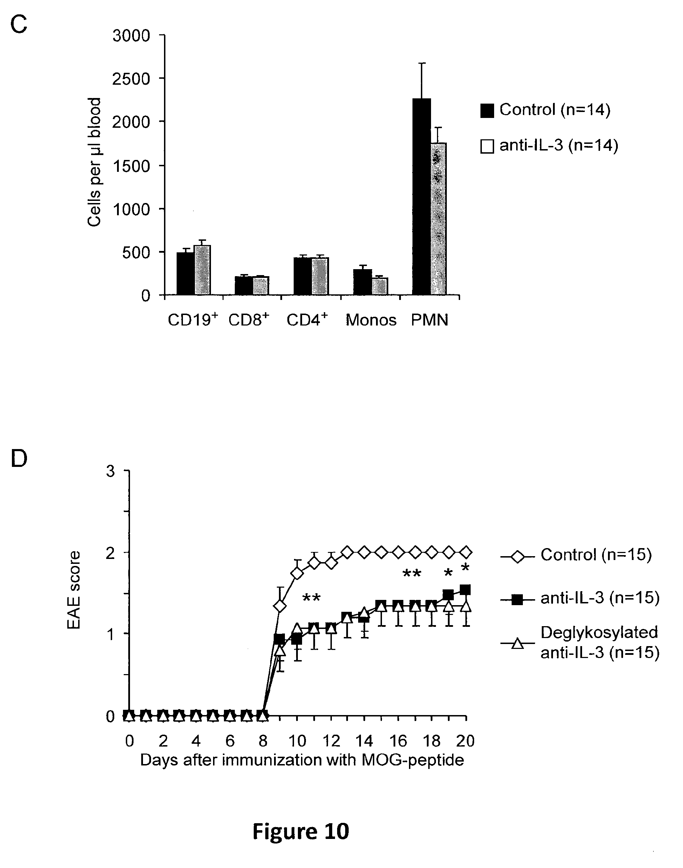

EAE was induced in C57BL/6 mice by immunization with MOG-peptide. IL-3 activity was blocked by daily i.p. injection of 50 µg of a neutralizing anti-IL-3 antibody (monoclonal antibody MP2-8F8). As control the same amount of purified rat IgG was injected. Treatment of mice was started immediately after immunization (day 0), on day 5 after immunization or 10 days after immunization, and continued until the penultimate day of the experiment (Figure 10: daily i.p. treatment with anti-IL-3 (50 µg) from day 0-19; Figure 11: daily i.p. treatment with anti-IL-3 (50 µg) from day 5-19; Figure 12: daily i.p. treatment with anti-IL-3 (50 µg) from day 10-19).

As shown in Fig. 10A, 11A and 12A, neutralization of IL-3 significantly reduced the development of clinical signs of EAE. When treatment was started on day 0, the severity of EAE symptoms was highly significantly reduced from day 10 to day 20. When treatment was started on day 5, there were significant reductions on day 10, 13 and 16. Inhibition of IL-3 from day 10-19 significantly reduced EAE symptoms on day 16-18. These data suggest that beneficial effects of anti-IL-3 antibodies become visible only after a couple of days and that treatment is more effective if started on day 0. As infiltration of cells into the brain occurs before clinical symptoms of EAE develop (i.e. before day 9) one can assume that IL-3 mediates early inflammatory and autoimmune processes in the brain.

It was further investigated whether the deglycosylated anti-IL-3 antibody is as efficient as the parental antibody. For that purpose, EAE was induced and mice were treated from day 0-19 with the deglycosylated MP2-8F8 anti-IL-3 antibody. As shown in Fig. 10D, the deglycosylated anti-IL-3 antibody prevents development of EAE as efficiently as the parental antibody, indicating that blockade of IL-3 is sufficient to suppress EAE, and depletion of IL-3 e.g. via Fc-receptor positive cells does not contribute to the *in vivo* activity of the IL-3 antibody.

In a further experiment, IL-3 levels were artificially increased by daily i.p. injection of recombinant IL-3 (200 ng) from day 5-21. In contrast to neutralization of IL-3, injection of recombinant IL-3 significantly exacerbated symptoms of EAE as seen on days 17-18 and 20-22 (Fig. 13A).

Flow cytometric quantification of cells infiltrating the brain showed a reduction of the number of total CD45⁺ leukocytes in mice treated with anti-IL-3 (Fig. 10B, 11B, 12B). In some cases there was also a reduction in infiltrating CD4⁺ T cell and CD11b⁺ monocytes. In contrast, treatment with recombinant IL-3 significantly increased the number of infiltrating total leukocytes, B cells and CD4⁺/CD8⁺ T cells (Fig. 13B).

At the end of the experiments, it was furthermore analyzed whether treatment with anti-IL-3 (from day 0) or IL-3 from day 5 alters the number of leukocyte subsets in the peripheral blood (Fig. 10C, 13C). No changes after treatment with IL-3 and a slight reduction in the number of monocytes after treatment with anti-IL-3 were observed. These data indicate that the number of cells infiltrating the brain and changes in EAE symptoms are not only a reflection of increased or decreased numbers of leukocyte subsets in the peripheral blood.

### Example 12

All methods mentioned in this example were carried out as described in Example 10.

EAE was induced in C57BL/6 mice by immunization with MOG-peptide on day 0. Mice were treated by daily i.p. injection of 50 µg of the neutralizing anti-IL-3 antibody MP2-8F8 (anti-IL-3) or 50 µg of purified rat IgG (Control) from day 0-10. Mice were analyzed in day 11. In addition, healthy C57BL/6 mice of same sex and age , kept in the same room without induction of EAE were analyzed (No EAE induced).

As shown in Fig 15. the number of leukocyte subpopulations infiltrating the brain was quantified by flow cytometry on day 11. CD45+ indicates the total number of CD45+ leukocytes, CD19+. CD8+, CD4+ and CD11b+ indicates the number of CD19+ B cells, CD8+ T cells, CD4+ T cells and CD11b+ monocytes respectively. Significance was calculated in relation to the control group. Mean +/- SEM.

These data indicate that the blockade of IL-3 with a monoclonal antibody reduces leukocyte infiltration of the brain.

The features of the present invention disclosed in the specification, the claims, and/or in the accompanying drawings may, both separately and in any combination thereof, be material for realizing the invention in various forms thereof.

## Claims

1. An anti-IL-3 antibody or an IL-3 binding fragment thereof for use in the treatment of an autoimmune disease selected from the group consisting of systemic lupus erythematosus and multiple sclerosis.

2. The anti-IL-3 antibody or IL-3 binding fragment thereof for use in the treatment of an autoimmune disease selected from the group consisting of systemic lupus erythematosus and multiple sclerosis according to claim 1, wherein said autoimmune disease is **characterized by** an increased plasma IL-3 level compared to a healthy state.

3. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody or said IL-3 binding fragment is administered to a patient in need thereof and wherein said patient is a human being.

4. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody is a monoclonal, polyclonal or chimeric antibody, or a combination thereof.

5. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody or said IL-3 binding fragment thereof is not immunogenic in a human subject.

6. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody or said IL-3 binding fragment thereof binds to the human IL-3 protein.

7. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody or said IL-3 binding fragment thereof binds to IL-3, preferably to human IL-3, with an affinity (K_{D}) of at least 10⁻⁵ M.

8. The anti-IL-3 antibody or IL-3 binding fragment thereof for use in the treatment of an autoimmune disease selected from the group consisting of systemic lupus erythematosus and multiple sclerosis according to any of the preceding claims, wherein binding of said anti-IL-3 antibody or said IL-3 binding fragment thereof to IL-3 prevents IL-3 from binding to and/or activating the interleukin-3 receptor.

9. The anti-IL-3 antibody or IL-3 binding fragment thereof for use in the treatment of an autoimmune disease selected from the group consisting of systemic lupus erythematosus and multiple sclerosis according to any of claims 3-8, wherein said anti-IL-3 antibody or said IL-3 binding fragment thereof is capable of decreasing the plasma level of unbound IL-3 in said patient upon administration of said antibody or fragment thereof to said patient.

10. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody or said IL-3 binding fragment thereof binds to a portion of the IL-3 amino acid sequence which portion consists of
a) residues 12-15 of the human IL-3 amino acid sequence (SEQ ID NO: 2),
b) residues 29-50 of the human IL-3 amino acid sequence (SEQ ID NO: 3),
c) the 18 most N-terminal amino acids of the human IL-3 amino acid sequence (SEQ ID NO: 4), or
d) the 22 most C-terminal amino acids of the human IL-3 amino acid sequence (SEQ ID NO: 5).

11. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody or said IL-3 binding fragment thereof competes with an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F 15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems
in a competitive binding assay.

12. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody or said IL-3 binding fragment thereof comprises an amino acid sequence that is identical to the amino acid sequence of the V_{H} region of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
or that has an amino acid sequence at least 90%, preferably at least 95%, more preferably at least 98%, more preferably at least 99% identical to the amino acid sequence of the V_{H} region of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
and/or
wherein said anti-IL-3 antibody or said IL-3 binding fragment thereof comprises an amino acid sequence that is identical to the amino acid sequence of the V_{L} region of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
or that has an amino acid sequence at least 90%, preferably at least 95%, more preferably at least 98%, more preferably at least 99% identical to the amino acid sequence of the V_{L} region of an antibody selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems.

13. The anti-IL-3 antibody or IL-3 binding fragment thereof for use in the treatment of an autoimmune disease selected from the group consisting of systemic lupus erythematosus and multiple sclerosis according to any of the preceding claims, wherein said anti-IL-3 antibody is selected from the group consisting of
- monoclonal anti-IL-3 antibody F14-570;
- monoclonal anti-IL-3 antibody F14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems,
and/or said IL-3 binding fragment thereof is an IL-3 binding fragment of an antibody selected from the group consisting of
- monoclonal anti-IL-3 antibody F14-570;
- monoclonal anti-IL-3 antibody F 14-746;
- monoclonal anti-IL-3 antibody F13-267;
- monoclonal anti-IL-3 antibody F15-216;
- monoclonal anti-IL-3 antibody Clone 11.14.6;
- monoclonal anti-IL-3 antibody Clone 13.4.4;
- monoclonal anti-IL-3 antibody Clone 4815 from R&D Systems; and
- monoclonal anti-IL-3 antibody Clone 4806 from R&D Systems.

14. The anti-IL-3 antibody or IL-3 binding fragment thereof for use in the treatment of an autoimmune disease selected from the group consisting of systemic lupus erythematosus and multiple sclerosis according to any of the preceding claims,
wherein, if said autoimmune disease is systemic lupus erythematosus, said anti-IL-3 antibody or said IL-3 binding fragment thereof is capable of reducing cellular infiltration in the kidney, reducing acute damage in the kidney, reducing chronic damage in the kidney, reducing immunoglobulin deposition in the kidney and reducing fibrosis in the kidney in said patient upon administration of said antibody or fragment thereof to said patient,
or if said autoimmune disease is multiple sclerosis, said anti-IL-3 antibody or said IL-3 binding fragment thereof is capable of reducing leukocyte infiltration in the brain, preferably during acute attacks, reducing demyelination and reducing axonal damage in said patient upon administration of said antibody or fragment thereof to said patient.

15. A pharmaceutical composition comprising at least one pharmaceutically acceptable carrier, diluent and/or excipient and an anti-IL-3 antibody or IL-3 binding fragment thereof as defined in any of the preceding claims for use in the treatment of an autoimmune disease selected from the group consisting of systemic lupus erythematosus and multiple sclerosis.
